# EUROPEAN PATENT SPECIFICATION

(11) **EP 2 868 297 B1**
(45) Date of publication and mention of the grant of the patent: **21.03.2018**
(21) Application number: 13808838.0
(22) Date of filing: 14.06.2013
(51) Int. Cl.: A61F 2/32, A61F 2/30, A61F 2/36

(54) **ARTIFICIAL JOINT STEM, ARTIFICIAL JOINT STEM COMPONENT, AND ARTIFICIAL JOINT STEM MANUFACTURING METHOD**
KÜNSTLICHER GELENKSCHAFT, KOMPONENTE FÜR EINEN KÜNSTLICHEN GELENKSCHAFT UND HERSTELLUNGSVERFAHREN FÜR EINEN KÜNSTLICHEN GELENKSCHAFT
TIGE D'ARTICULATION ARTIFICIELLE, COMPOSANT DE TIGE D'ARTICULATION ARTIFICIELLE, ET PROCÉDÉ DE FABRICATION DE TIGE D'ARTICULATION ARTIFICIELLE

(30) Priority: 29.06.2012 JP 2012146362
(43) Date of publication of application: 06.05.2015
(73) Proprietor: KYOCERA Corporation, Kyoto City (JP)
(72) Inventor: SHIMOZONO,Takayoshi, Osaka-shi, Osaka 532-0003 (JP); WAKIYAMA,Miyo, Osaka-shi, Osaka 532-0003 (JP)
(74) Representative: Isarpatent
(86) International application number: PCT/JP2013/066470
(87) International publication number: WO 2014/002795

(56) References cited:
- EP-A1- 1 958 650
- WO-A1-2010/019807
- JP-A- H0 690 972
- JP-A- S6 340 547
- JP-A- H01 305 944
- JP-A- 2001 245 911
- JP-B2- 4 911 566
- US-A1- 2007 150 068

## Description

### Technical Field

The present invention relates to an artificial joint stem used in an operation for replacing a patient's joint with an artificial joint, as well as an artificial joint stem component and an artificial joint stem manufacturing method.

### Background Art

Conventionally, patients deemed to have a joint abnormality have undergone an artificial joint replacement operation in which a portion or all of the joint is replaced with an artificial joint. As one type of an artificial joint, an artificial hip joint has an acetabular component and a femur-side artificial joint stem (e.g., see Patent Document 1). A recessed shell and a liner, which are the acetabular components, are attached to the pelvic acetabulum. Also, the femur-side artificial joint stem (stem) is formed with an elongated shape, and is inserted into the medullary cavity of the patient's femur.

A bone head ball is fixed to the neck portion of the stem. The bone head ball is arranged inside the liner. According to this configuration, the bone head ball slides against the liner in conjunction with a movement of the femur and the stem.

In an artificial hip joint replacement operation, for example, the bone head at the proximal portion of the patient's femur is resected, and then the stem is inserted into the medullary cavity of the femur. The stem is fixed to the proximal portion of the femur.

The stem disclosed in Patent Document 1 has a femoral stem and porous bodies. The femoral stem is made of a titanium alloy. Multiple recessions are formed in the surface of the proximal portion of the femoral stem. The recessions are arrayed with spaces therebetween in the circumferential direction of the surface of the proximal portion of the stem. The porous bodies are fitted into respective recessions. Accordingly, in the proximal portion of the stem, multiple porous bodies are arrayed with spaces therebetween in the circumferential direction of the stem body. The porous bodies are each manufactured by stacking metal plates that have a thickness of 150 µm or less and have a large number of holes formed therein. The porous bodies are configured so as to fit into respective recessions. Specifically, the porous bodies are formed with a flat plate shape or a bow-like curved plate shape. The porous bodies are fitted into corresponding recessions of the femoral stem.

The stem is inserted into the medullary cavity of the femur, and then after a certain period has elapsed, bone tissue in the proximal portion of the femur penetrates into the porous bodies of the stem. The femoral stem is fixed to the femur in this way.

### Citation List

### Patent Document

Patent Document 1: JP H6-7388A ([Claim 1], [0093] to [0104], FIGS. 36 to 43)

The following further patent documents disclose further technological background information for the present invention:
- D1: EP 1 958 650 A1 (MITSUBISHI MATERIALS CORP [JP]) 20 August 2008 (2008-08-20)
- D2: WO 2010/019807 A1 (SMED TA TD LLC [US]; NEBOSKY PAUL S [US]; ZIMMERMAN SARAH L [US]; STAL) 18 February 2010 (2010-02-18)
- D3: US 2007/150068 A1 (DONG NICHOLAS N G [US] ET AL) 28 June 2007 (2007-06-28)

Document D1 relates to the preamble of claims 1, 11, 12. Document D2 describes an implant with spatially varying porosity made up in a layered structure in which a thickness of one or more layers may be varied medially or laterally. One layer can also taper. Document D3 related to a gradient porous implant including a porous metal foam or foam-like structure having pores defined by metal struts or webs wherein the porous structure has directionally controlled pore characteristics.

### Disclosure of the Invention

### Problem to be Solved by the Invention

As previously described, with the configuration disclosed in Patent Document 1, multiple recessions need to be formed in the surface of the proximal portion of the stem body. The stem body is made of a titanium alloy, and it is difficult to form the recessions. The manufacturing of the stem body is therefore laborious. Also, the recessions are configured such that flat plate shaped porous bodies are fitted therein. The peripheries of the bottom portions of the recessions therefore have an angular shape, and stress is likely to become concentrated there. For this reason, if load from the femur acts on the recessions of the stem body via the porous bodies for example, the amount of stress generated in the recessions will increase. Moreover, since multiple recessions are formed in the stem body, stress concentration occurs at many locations in the stem body. This stress concentration is not preferable in terms of raising the allowable load of the femoral stem.

Also, the same number of porous bodies as the number of recessions need to be provided, and the shapes of the porous bodies need to be different from each other. The manufacturing of the porous bodies is therefore laborious. Moreover, the porous bodies need to be separately fitted into the recessions. The task of bonding the porous bodies and the stem body is therefore laborious, and the manufacturing of the femoral stem requires a large amount of labor.

Also, the porous bodies are constructed by stacking metal plates. It is therefore difficult to form porous bodies with a complex shape such as a shape in which the thickness changes in a continuous manner, and the degree of freedom in the shape is low.

In light of the aforementioned circumstances, an object of the present invention is to provide an artificial joint stem with which the amount of labor required in manufacturing is reduced, the strength can be increased through the suppression of stress concentration, and the degree of freedom in the shape is high, as well as an artificial joint stem component and an artificial joint stem manufacturing method.

### Means for Solving the Problem

This object is solved by an artificial joint stem of claim 1, by an artificial joint stem component of claim 11 and by a method of manufacturing an artificial joint stem of claim 12. Further advantageous improvements and embodiments of the invention are listed in the dependent claims. Hereinafter, some aspects of the invention are highlighted before coming to a detailed description of the embodiments.

An artificial joint stem according to a first aspect of the invention for achieving the above object includes: a stem body portion; and a porous portion that has a porous body and is bonded to the stem body portion, wherein the stem body portion has an insertion portion arranged to be inserted into a medullary cavity of a bone of a biological body, and the porous portion is a tubular standalone portion formed by stacking layers of a biocompatible metal powder, and is arranged so as to surround a proximal portion of the insertion portion.

According to this first aspect of the invention, the porous portion is provided as a tubular standalone portion. For this reason, the stem body portion can be given a simple shape for bonding with the porous portion. Accordingly, the stem body portion has few or no portions that require processing for bonding with the porous portion, and it is possible to reduce the amount of labor required for processing the stem body portion. In particular, in the case where the stem body portion is made of a titanium alloy, the amount of labor for processing the stem body portion can be significantly reduced. Also, as described above, the stem body portion can be given a simple shape for bonding with the porous portion. For this reason, the stem body portion can have few or no portions where stress concentration occurs due to the shape for bonding with the porous portion. In this way, as a result of suppressing stress concentration in the stem body portion, the strength of the artificial joint stem can be raised even higher.

Also, the porous portion is provided as a standalone portion. Accordingly, the task of bonding the porous portion to the stem body portion can be performed in a single task. This makes it possible to reduce the amount of labor required in manufacturing the artificial joint stem.

Also, the porous portion is formed by stacking layers of a metal powder. According to this configuration, even if the porous portion has a complex shape, the porous portion can be easily manufactured by changing how the metal powder layers are stacked. For this reason, it is possible to raise the degree of freedom in the shape of the porous portion, thus enabling raising the degree of freedom in the shape of the artificial joint stem.

Therefore, according to the present invention, it is possible to provide an artificial joint stem with which the amount of labor required in manufacturing is reduced, the strength can be increased through the suppression of stress concentration, and the degree of freedom in the shape is high.

An artificial joint stem according to a second aspect of the invention is the artificial joint stem of the first aspect of the invention, wherein the thickness of a proximal portion of the porous body is set larger than the thickness of a distal portion of the porous body.

According to this second aspect of the invention, in the state where the artificial joint stem has been inserted into the medullary cavity of a bone of a biological body, the proximal portion of the porous body is arranged at a relatively shallow depth from the opening of the hole portion. When the distal portion of the artificial joint stem is fixed to the femur, the proximal portion of the stem easily becomes wobbly. Increasing the thickness of the proximal portion of the porous body makes it possible for more of the bone tissue of the biological body to penetrate into the proximal portion. Accordingly, it is possible to ensure sufficient bonding force between the bone and the proximal portion of the porous portion near the opening of the medullary cavity of the bone. Also, the distal portion of the porous body is arranged at a relatively deep depth from the opening of the medullary cavity. Reducing the thickness of the distal portion makes it possible to suppress the case where the bone tissue of the biological body penetrates more than necessary into the distal portion. This makes it possible to suppress stress shielding. In other words, load from the artificial joint stem can be transmitted in a balanced manner to the portion of the proximal portion of the bone that faces the porous portion. As a result, it is possible to suppress bone atrophy in the proximal portion of the bone, thus making it possible for the state in which the artificial joint stem and the bone are strongly bonded to be maintained over a long period.

An artificial joint stem according to a third aspect of the invention is the artificial joint stem of the first or second aspect of the invention, wherein the porous body is configured such that the porosity of the porous body decreases from the proximal portion side of the porous body toward the distal portion side of the porous body.

According to this invention, in the state where the artificial joint stem has been inserted into the medullary cavity of a bone of a biological body, more of the bone tissue of the biological body can penetrate into the proximal portion side of the porous body. Accordingly, it is possible to ensure sufficient bonding force between the bone and the proximal portion of the porous body near the opening of the medullary cavity of the bone. Also, the distal portion of the porous body is arranged at a relatively deep depth from the opening of the medullary cavity. Reducing the porosity of the distal portion side makes it possible to suppress the case where the bone tissue of the biological body penetrates more than necessary into the distal portion. This makes it possible to more reliably suppress stress shielding.

An artificial joint stem according to a fourth aspect of the invention is the artificial joint stem of any of the first to third aspects of the invention, wherein a distal end portion of the porous portion is a dense body.

According to this configuration, the distal end portion of the porous portion, for example, can be formed as a dense body similar to the stem body portion. Since bone tissue cannot penetrate into the dense body, the dense body and the bone do not become bonded to each other. Accordingly, load is more easily transmitted in the proximal portion, and stress shielding can be further suppressed.

An artificial joint stem according to a fifth aspect of the invention is the artificial joint stem of any of the first to fourth aspects of the invention, wherein the porous portion is formed in an integrated state by successively stacking and temporary melting layers of the metal powder.

According to this invention, the porosity of the porous body can be easily adjusted by changing the degree of melting metal powder, thus making it possible to further raise the degree of freedom in manufacturing the porous portion.

An artificial joint stem according to a sixth aspect of the invention is the artificial joint stem of any of the first to fifth aspects of the invention, wherein the stem body portion and the porous portion are formed using members that are separate from each other, and are integrally bonded to each other.

According to this invention, the stem body portion and the porous portion can be manufactured separately. For example, the stem body portion can be formed by casting, and the porous portion can be formed by melting a metal powder. Accordingly, the degree of freedom in manufacturing the artificial joint stem can be increased. Also, the air hole portions in the porous body can be manufactured more precisely than in the case where the stem body portion and the porous portion are formed together.

An artificial joint stem according to a seventh aspect of the invention is the artificial joint stem of the sixth aspect of the invention, wherein the stem body portion and the porous portion are bonded to each other by bonding using a slurry or bonding using diffusion joining.

According to this invention, the stem body portion and the porous portion can be bonded to each other using a simple method.

An artificial joint stem according to an eighth aspect of the invention is the artificial joint stem of the seventh aspect of the invention, wherein the stem body portion and the porous portion are bonded to each other by bonding using the slurry, the porous portion has an opposing surface that opposes the proximal portion of the insertion portion, and a plurality of hole portions are formed in the opposing surface.

According to this invention, the binder component in the slurry can be allowed to escape through the hole portions when bonding the stem body portion and the porous portion by heating. Accordingly, it is possible to suppress the formation of cavities due to the binder component gas between the stem body portion and the porous portion, and it is possible to suppress variation in the bonding force between the stem body portion and the porous portion.

An artificial joint stem according to a ninth aspect of the invention is the artificial joint stem of the eighth aspect of the invention, wherein at least one condition is satisfied among conditions that the diameter of each of the hole portions is 600 µm or less, the total area ratio of the plurality of hole portions in the opposing surface is 30% or less, and the distance between adjacent hole portions is 0.5 to 7.0 mm.

According to this invention, the diameter of each of the hole portions is 600 µm or less, thus making it possible to suppress the case where the liquid component of the slurry leaks into and obstructs the hole portions in the outer peripheral surface of the porous body, thereby enabling ensuring sufficient bonding force between the bone and the porous portion. Also, at least one condition is satisfied out of the conditions that the area ratio is 30% or less and the distance between adjacent hole portions is 0.5 to 7.0 mm, thus making it possible to efficiently discharge the binder component in the slurry to the outside of the artificial joint stem, while ensuring sufficient bonding force between the stem body portion and the porous portion.

An artificial joint stem according to a tenth aspect of the invention is the artificial joint stem of any of the first to ninth aspects of the invention, wherein a positioning step portion for defining the position of the porous portion relative to the stem body portion is formed in a proximal portion of the insertion portion.

According to this invention, the position of the porous portion relative to the position of the stem body portion can be defined more accurately.

An artificial joint stem component according to an eleventh aspect of the invention is an artificial joint stem component that is a component to be bonded to a stem body portion to be inserted into a medullary cavity of a bone of a biological body, the artificial joint stem component including: a porous portion that has a porous body and is to be bonded to the stem body portion, wherein the porous portion is a tubular standalone component formed by stacking layers of a biocompatible metal powder.

According to this invention, the porous portion is provided as a tubular standalone portion. For this reason, the stem body portion can be given a simple shape for bonding with the porous portion. Accordingly, the stem body portion has few or no portions that require processing for bonding with the porous portion, and it is possible to reduce the amount of labor required for processing the stem body portion. In particular, in the case where the stem body portion is made of a titanium alloy, the amount of labor for processing the stem body portion can be significantly reduced. Also, as described above, the stem body portion can be given a simple shape for bonding with the porous portion. For this reason, the stem body portion can have few or no portions where stress concentration occurs due to the shape for bonding with the porous portion. In this way, as a result of suppressing stress concentration in the stem body portion, the strength of the artificial joint stem can be raised even higher.

Also, the porous portion is provided as a standalone portion. Accordingly, the task of bonding the porous portion to the stem body portion can be performed in a single task. This makes it possible to reduce the amount of labor required in manufacturing the artificial joint stem.

Also, the porous portion is formed by stacking layers of a metal powder. According to this configuration, even if the porous portion has a complex shape, the porous portion can be easily manufactured by changing how the metal powder layers are stacked. For this reason, it is possible to raise the degree of freedom in the shape of the porous portion, thus enabling raising the degree of freedom in the shape of the artificial joint stem.

Therefore, according to the present invention, it is possible to provide an artificial joint stem component with which the amount of labor required in manufacturing is reduced, the strength of the artificial joint stem can be increased through the suppression of stress concentration, and the degree of freedom in the shape is high.

A method of manufacturing an artificial joint stem according to a twelfth aspect of the invention is a method of manufacturing an artificial joint stem that has a stem body portion having an insertion portion that is to be inserted into a medullary cavity of a bone of a biological body, and a porous portion that has a porous body and is bonded to the stem body portion, the method including: a stem body portion formation step of forming the stem body portion; a porous portion formation step of forming the porous portion; and a bonding step of bonding the stem body portion to the porous portion, wherein in the porous portion formation step, the porous portion is formed by forming a tubular standalone component by stacking layers of a biocompatible metal powder, and in the bonding step, the porous portion is bonded to the stem body portion in a state in which the porous portion is mated with a proximal portion of the insertion portion.

According to this invention, the porous portion is provided as a tubular standalone portion. For this reason, the stem body portion can be given a simple shape for bonding with the porous portion. Accordingly, in the stem body portion formation step, there are few or no portions that require processing for bonding with the porous portion, and it is possible to reduce the amount of labor required for processing the stem body portion. In particular, in the case where the stem body portion is made of a titanium alloy, the amount of labor for processing the stem body portion can be significantly reduced. Also, as described above, the stem body portion can be given a simple shape for bonding with the porous portion. For this reason, the stem body portion can have few or no portions where stress concentration occurs due to the shape for bonding with the porous portion. In this way, as a result of suppressing stress concentration in the stem body portion, the strength of the artificial joint stem can be raised even higher.

Also, the porous portion is provided as a standalone portion. Accordingly, in the bonding step, the task of bonding the porous portion to the stem body portion can be performed in a single task. This makes it possible to reduce the amount of labor required in manufacturing the artificial joint stem.

Also, in the porous portion formation step, the porous portion is formed by stacking layers of a metal powder. According to this configuration, even if the porous portion has a complex shape, the porous portion can be easily manufactured by changing how the metal powder layers are stacked. For this reason, it is possible to raise the degree of freedom in the shape of the porous portion, thus enabling raising the degree of freedom in the shape of the artificial joint stem.

Therefore, according to the present invention, it is possible to provide a method of manufacturing an artificial joint stem with which the amount of labor required in manufacturing is reduced, the strength can be increased through the suppression of stress concentration, and the degree of freedom in the shape is high.

A method of manufacturing an artificial joint stem according to a thirteenth aspect of the invention is the method of manufacturing an artificial joint stem of the twelfth aspect of the invention claim 12, wherein in the bonding step, the porous portion is bonded to the insertion portion so as to constrict the proximal portion of the insertion portion.

According to this invention, the porous portion and the insertion portion can be bonded so as to form an interference fit. This makes it possible to further increase the bonding force between the porous portion and the insertion portion. It is also possible to suppress the case where the porous portion becomes separated from the insertion portion during the task of bonding the porous portion and the insertion portion.

A method of manufacturing an An artificial joint stem according to the fourteenth aspect of the invention is the method of manufacturing an artificial joint stem of the twelfth or thirteenth aspect of the invention, wherein in the bonding step, the porous portion and the insertion portion are bonded by interposing a slurry between the porous portion and the insertion portion and heating the slurry, or by performing diffusion joining on the porous portion and the insertion portion.

According to this invention, the stem body portion and the porous portion can be bonded to each other using a simple method.

### Effects of the Invention

According to the present invention, it is possible to provide an artificial joint stem with which the amount of labor required in manufacturing is reduced, the strength can be increased through the suppression of stress concentration, and the degree of freedom in the shape is high, as well as an artificial joint stem component and an artificial joint stem manufacturing method.

### Brief Description of the Drawings

FIG. 1 is a partial cross-sectional diagram showing an artificial hip joint that includes an artificial hip joint component according to an embodiment of the present invention.
FIG. 2 is a side view of a stem.
FIG. 3 is a cross-sectional view of a porous portion of the stem, in a state in which the stem is viewed from the side.
FIG. 4 is a cross-sectional view of the porous portion on its own.
FIG. 5(a) is an enlarged schematic side view of a portion of the inner peripheral surface of the porous portion, and FIG. 5(b) is an enlarged schematic side view of a portion of the outer peripheral surface of the porous portion.
FIG. 6 is a flowchart for describing stem manufacturing steps.
FIG. 7 is a diagram for describing main points in stem manufacturing.
FIG. 8 is a cross-sectional diagram showing a main portion of a variation of the present invention.
FIG. 9 is a cross-sectional diagram showing a main portion of another variation of the present invention.

### Description of Embodiments

Hereinafter, modes for carrying out the present invention will be described with reference to the drawings. Note that the present invention is broadly applicable as an artificial joint component used in an operation for replacing a patient's joint with an artificial joint.

### Overview of configuration of artificial hip joint

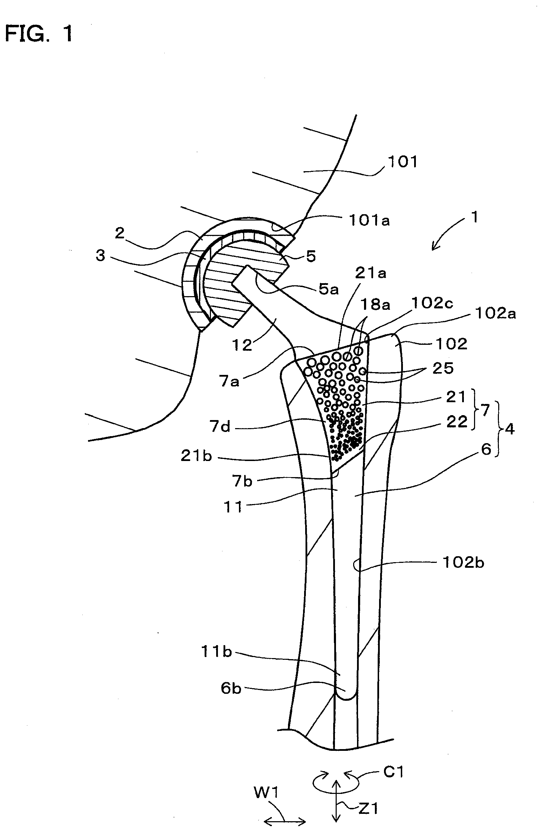

FIG. 1 is a partial cross-sectional diagram showing an artificial hip joint 1 that includes an artificial joint component according to an embodiment of the present invention. FIG. 1 shows a state in which the artificial hip joint 1 has been attached to a patient, and shows a portion of a pelvis 101 and a portion of a femur 102. As shown in FIG 1, the artificial hip joint 1 is provided as an artificial joint for allowing relative displacement of the femur 102 relative to an acetabulum 101a of the pelvis 101. Note that unless stated otherwise, the following description is given based on the state in which the artificial hip joint 1 has been attached to the pelvis 101 and the femur 102.

The artificial hip joint 1 is configured so as to include a shell 2 and a liner 3 that are attached to the acetabulum 101a of the pelvis 101, and a stem 4 and a bone head ball 5 that are attached to the femur 102.

The shell 2 and the liner 3 are supported to the pelvis 101 and work in cooperation with the bone head ball 5 to form a spherical joint, and allow movement of the femur 102 relative to the pelvis 101. The shell 2 is a cup-shaped member that has a recession, and is fixed to the acetabulum 101a of the pelvis 101. The liner 3 is fixed to the shell 2.

The liner 3 is formed using a synthetic resin, a metal, a ceramic, or the like. The liner 3 is a cup-shaped member that has a recession. The bone head ball 5 is in slidable contact with the inner surface of the liner 3.

The bone head ball 5 is approximately ball-shaped. Examples of the material for the bone head ball 5 include a metal material such as a cobalt-chromium alloy or stainless steel, a polymer material such as polyethylene, and a ceramic material such as alumina or zirconia, all of which have received approval as a medical device for biological implantation. An insertion hole 5a is formed in the bone head ball 5. The insertion hole 5a extends from the surface of the bone head ball 5 to the interior of the bone head ball 5, and receives insertion of a later-described neck portion 12 of the stem 4.

Note that although the artificial hip joint is described in the present embodiment taking the example of a configuration in which the bone head ball 5 is connected to the stem 4, this need not be the case. For example, the bone head ball 5 may be formed integrally with the stem 4. Also, the shell 2 does not need to be provided.

The stem 4 is provided as a portion that supports the bone head ball 5 and is fixed to the patient's femur 102.

According to the above configuration, the femur 102 is displaced relative to the acetabulum 101a due to the bone head ball 5 sliding against the inner surface of the liner 3.

### Detailed configuration of stem

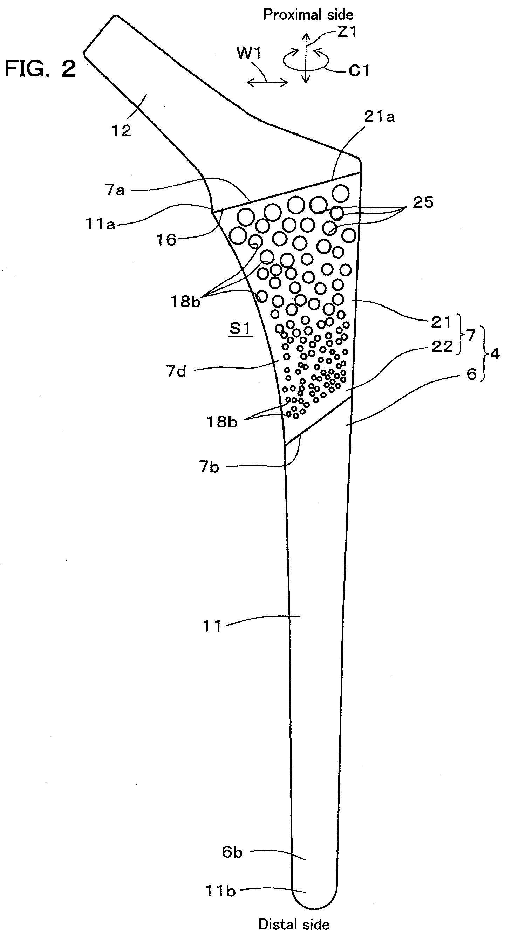

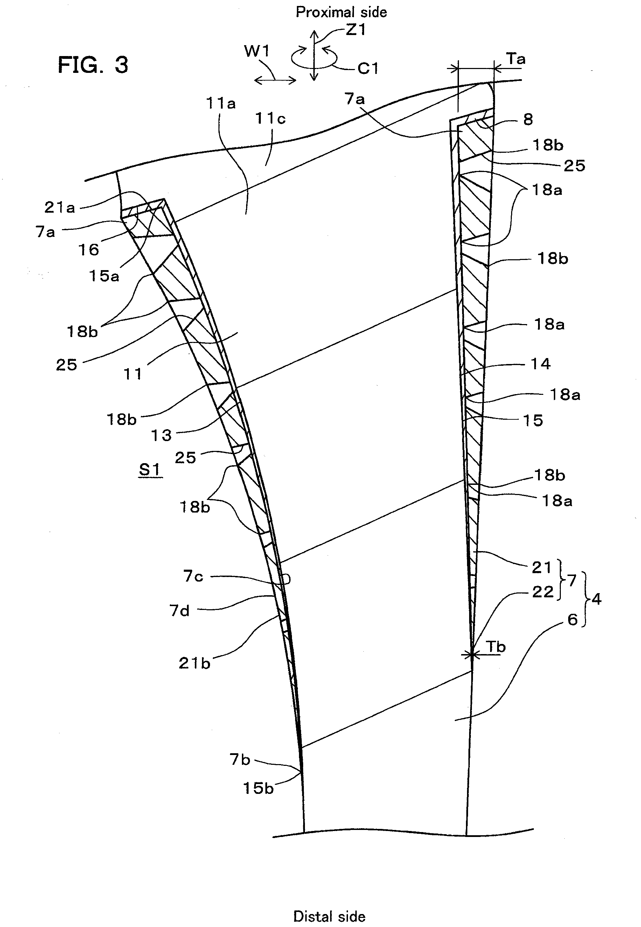

FIG. 2 is a side view of the stem 4. FIG. 3 is a cross-sectional view of a porous portion of the stem 4, in which the stem 4 is viewed from the side. As shown in FIGS. 1, 2, and 3, the stem 4 is provided as an artificial joint stem. Examples of the material for the stem 4 include a biocompatible metal material that has received approval as a medical device for biological implantation, such as titanium, a titanium alloy (e.g., Ti-6Al-4V or Ti-6Al-2Nb-1Ta), a cobalt alloy (e.g., a cobalt-chromium alloy), and stainless steel. The material for the stem 4 may be one or more of the above-illustrated types of metal materials, or may be a biocompatible metal material made up of one or more types of metal materials other than those illustrated above.

In the present embodiment, the stem 4 is configured as a single component. The stem 4 is formed with an elongated plate shape (stem shape), and an intermediate portion of the stem 4 has a curved shape.

The stem 4 includes a stem body portion 6, a porous portion 7, and a bonding layer 8. In the present embodiment, the stem body portion 6 and the porous portion 7 are formed as members that are separate from each other. Also, the stem body portion 6 and the porous portion 7 are integrally bonded to each other via the bonding layer 8.

In the present embodiment, the stem body portion 6 is a dense body formed using any of the above-described metal materials. In the present embodiment, "dense body" refers to a portion in which substantially no air holes are provided, such as a portion having a porosity of less than several percent (including zero percent). In other words, in the present embodiment, the stem body portion 6 is not provided with a porous structure.

The stem body portion 6 is provided as a portion that makes up a majority of the stem 4. In the present embodiment, the stem body portion 6 is formed by casting. Specifically, the stem body portion 6 is formed by a material such as any of the above-described metal materials given as an example of the material for the stem 4 being melted and poured into a mold.

Note that the stem body portion 6 may be formed by performing machining such as cutting on a lump of any of the above-described metal materials. Also, the stem body portion 6 may be formed by performing forging on a lump of any of the above-described metal materials, and then performing machining such as cutting. Also, the stem body portion 6 may be formed by a later-described stacking and shaping method, or may be formed by sintering. The surface of the stem body portion 6 is formed as a smooth surface. Examples of processing methods for forming such a surface include machining such as grinding and polishing.

The stem body portion 6 has an insertion portion 11 and a neck portion 12.

The insertion portion 11 is configured so as to be inserted into a medullary cavity 102b of the femur 102. Note that it is sufficient that at least a portion of the insertion portion 11 is inserted into the medullary cavity 102b, and there is no need for the entirety of the insertion portion 11 to be inserted into the medullary cavity 102b.

In the present embodiment, the thickness of the stem body portion 6 (the length of the stem body portion 6 in terms of the direction orthogonal to the paper plane in FIGS. 1 to 3) is set approximately constant. Note that the thickness of the stem body portion 6 may decrease in a continuous manner or in a stepwise manner from a proximal portion 11a of the insertion portion 11 toward a distal portion 11b.

The insertion portion 11 extends along a predetermined lengthwise direction Z1 that conforms to the lengthwise direction of the femur 102. In the present embodiment, the insertion portion 11 has an elongated shape that tapers off from the proximal portion 11a of the insertion portion 11 toward the distal portion 11b. Note that the insertion portion 11 does not need to have a tapered shape. Also, in the present embodiment, the lengthwise direction Z1 of the stem 4 is also the lengthwise direction of the insertion portion 11.

The proximal portion 11a of the insertion portion 11 is provided as a portion that is continuous with the neck portion 12. Also, the proximal portion 11a is provided as a portion that receives load from a proximal portion 102a of the femur 102 via the porous portion 7. The proximal portion 11a forms a portion of the insertion portion 11 in the lengthwise direction Z1. In the proximal portion 11a, a proximal end portion 11c is surrounded by an opening 102c of the medullary cavity 102b. The proximal portion 11a is shaped such that the length in a width direction W1 orthogonal to the lengthwise direction Z1 decreases in a continuous manner as the proximal portion 11a extends toward the distal portion 11b along the lengthwise direction Z1.

More specifically, the proximal portion 11a has a pair of side portions 13 and 14 that face each other in a width direction X1. The one side portion 13 is formed with a curved shape so as to recede toward the other side portion 14 side. The other side portion 14 extends approximately parallel with the lengthwise direction Z1. Accordingly, the proximal portion 11a is shaped so as to increase in width as it extends toward the neck portion 12. Note that the other side portion 14 may be formed with a curved shape likewise to the one side portion 13, or may extend with an inclination relative to the lengthwise direction Z1 in a lateral view.

The proximal portion 11a has a mating portion 15. The mating portion 15 is provided as a portion for mating with the porous portion 7. The mating portion 15 is arranged over approximately the entire range of the proximal portion 11a. The mating portion 15 is formed so as to be elongated in the width direction W1 in a cross-section orthogonal to the lengthwise direction Z1 (not shown). The mating portion 15 is provided over the entire range of the proximal portion 11a in a circumferential direction C1 (the direction around the axis extending in the lengthwise direction Z1).

The surface of the mating portion 15 is a smooth surface formed by using surface processing such as grinding or polishing. A proximal end portion 15a and a distal end portion 15b of the mating portion 15 each extend toward one side in the lengthwise direction Z1 so as to approach the neck portion 12 from the one side portion 13 toward the other side portion 14. A positioning step portion 16 is continuous with the proximal end portion 15a of the mating portion 15.

The positioning step portion 16 is provided for defining the position of the porous portion 7 relative to the stem body portion 6. The position of the porous portion 7 is determined due to the porous portion 7 coming into contact with the positioning step portion 16. The positioning step portion 16 extends so as to protrude from the mating portion 15 toward the outer side of the stem body portion 6. In the present embodiment, the positioning step portion 16 is formed over the entire range of the stem body portion 6 in the circumferential direction C1. The amount of protrusion of the positioning step portion 16 from the mating portion 15 is set to be approximately the same as a thickness Ta of the porous portion 7 in a proximal end portion 7a of the porous portion 7. The distal portion 11b of the insertion portion 11 is arranged in the extending direction of the lengthwise direction Z1 with respect to the mating portion 15.

Although the distal portion 11b of the insertion portion 11 is inserted into the medullary cavity 102b, the distal portion 11b is provided as a portion that is not fixed to the femur 102. A large amount of insertion of the insertion portion 11 into the medullary cavity 102b can be ensured by inserting the distal portion 11b of the insertion portion 11 into the medullary cavity 102b. Accordingly, the stem 4 is inserted to a more ideal position in the medullary cavity 102b. The distal portion 11b of the insertion portion 11 directly faces the medullary cavity 102b. However, as previously mentioned, the surface of the insertion portion 11 is a smooth surface and does not bond with the bone tissue of the medullary cavity 102b. In the present embodiment, the length of the distal portion 11b of the insertion portion 11 in the lengthwise direction Z1 is set larger than the length of the proximal portion 11a of the insertion portion 11. The neck portion 12 extends from the insertion portion 11 having the above configuration.

The neck portion 12 is provided as a portion that protrudes from the proximal portion 11a of the insertion portion 11 toward the liner 3. The neck portion 12 protrudes from the medullary cavity 102b. The neck portion 12 extends with an inclination relative to the lengthwise direction Z1. The neck portion 12 is formed with a tapered shape. The neck portion 12 is inserted into the insertion hole 5a of the bone head ball 5. The neck portion 12 and the bone head ball 5 are fixed by press-fitting such that the bone head ball 5 does not become separated from the neck portion 12. The stem body portion 6 configured as described above is bonded to the porous portion 7.

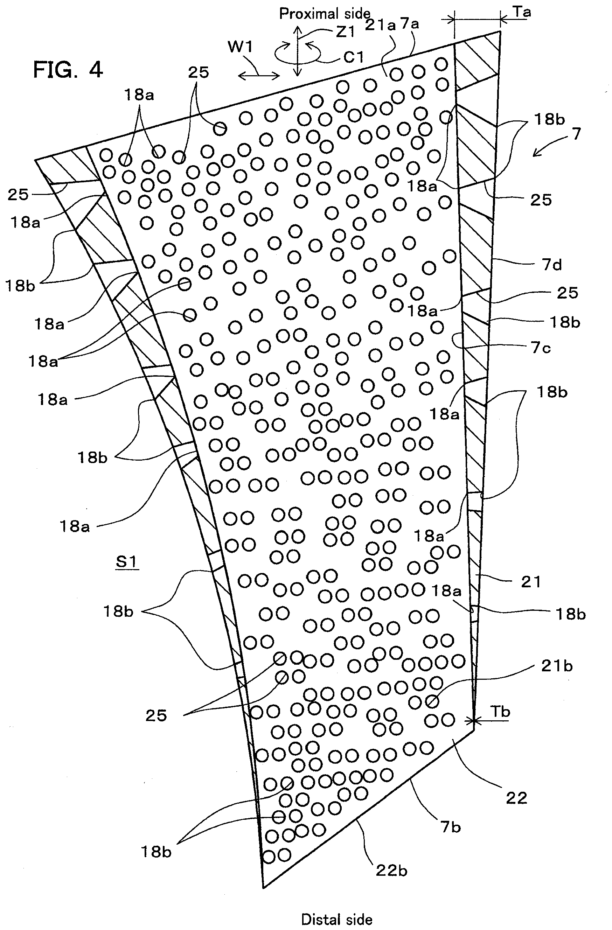

FIG. 4 is a cross-sectional view of the porous portion 7 on its own. As shown in FIGS. 1 to 4, the porous portion 7 is provided for mechanical bonding (fixing) with the medullary cavity 102b in the proximal portion 102a of the patient's femur 102. As previously mentioned, the porous portion 7 is formed using a member separate from the stem body portion 6, and exists as a tubular standalone portion in a standalone state. In the present embodiment, the porous portion 7 is formed using a stacking and shaping method. In this stacking and shaping method, the same metal powder as the material for the stem 4 is stacked in layers of a predetermined thickness, and the stacked layer of metal powder is melted temporary each time the stacking is performed, thus forming the porous portion 7. The stem 4 is formed by bonding the porous portion 7 to the stem body portion 6 using the bonding layer 8.

The porous portion 7 is formed with a cylindrical shape that is elongated in the lengthwise direction Z1, and is mated with the mating portion 15 of the stem body portion 6. In other words, the porous portion 7 is attached to the proximal portion 11a of the insertion portion 11. According to this configuration, the porous portion 7 is arranged so as to surround the proximal portion 11a of the insertion portion 11. At least a portion of the porous portion 7 is inserted into the medullary cavity 102b. In the present embodiment, the entirety of the porous portion 7 is inserted into the medullary cavity 102b.

The porous portion 7 forms a portion of the outer surface of the stem 4 and comes into direct contact with the proximal portion 102a of the femur 102. In the present embodiment, the porous portion 7 is formed such that the length in the lengthwise direction Z1 decreases from the one side portion 13 of the insertion portion 11 toward the other side portion 14. Note that the length of the porous portion 7 in the lengthwise direction Z1 may be constant over the entire range in the circumferential direction C1.

The porous portion 7 has a porous body 21 and a dense body 22.

The porous body 21 configures the portion of the porous portion 7 other than the distal end portion 7b of the porous portion 7. The porous body 21 is provided as a surface modification portion, and is provided for strong bonding with the proximal portion 102a of the femur 102. Specifically, the porous body 21 has a porous structure having a predetermined porosity. The porous body 21 is formed by setting the amount of melting of the metal powder serving as the material for the porous portion 7 to a predetermined amount or less, and has a large number of air holes 25. In the present embodiment, the porosity is defined as the value obtained by dividing the air hole (space) volume B per unit volume A by the unit volume A and multiplying the resulting value by 100. This can be expressed as porosity=(B/A)×100.

In the present embodiment, the porosity of the porous body 21 decreases from the proximal portion 21a of the porous body 21 toward the distal portion 21b side. The porosity of the porous body 21 may decrease in a continuous manner toward the distal end portion 7b side of the porous portion 7, or may decrease in a stepwise manner.

Also, in the proximal end portion 7a of the porous portion 7, the thickness (wall thickness) Ta of the porous portion 7 is set larger than the thickness Tb of the porous portion 7 in the distal end portion 7b of the porous portion 7 (Ta>Tb). In the present embodiment, the thickness of the porous portion 7 decreases in a continuous manner from the proximal end portion 7a of the porous portion 7 toward the distal end portion 7b side. Accordingly, the thickness of the distal portion 21b of the porous body 21 is smaller than the thickness of the proximal portion 21a of the porous body 21.

Bone tissue in the proximal portion 102a of the femur 102 can penetrate into the air holes 25 of the porous body 21. Accordingly, the porous body 21 bonds with the proximal portion 102a and is strongly bonded to the femur 102. Also, the amount of penetration of the bone tissue of the proximal portion 102a into the porous body 21, that is to say the bonding force between the femur 102 and respective portions of the porous body 21 increases as the thickness of the respective portions increases. Also, it is preferable that the porosity of the porous body 21 is 30 to 60%, and that the pore diameter is 100 to 300 µm. Optimizing the porosity and the pore diameter of the porous body 21 makes it possible to maximize the bonding force between the femur 102 and respective portions of the porous body 21.

The portion of the porous body 21 that is adjacent to the positioning step portion 16 of the stem body portion 6, that is to say the proximal end portion 7a, comes into contact with the positioning step portion 16. Accordingly, the position of the porous portion 7 relative to the stem body portion 6 is defined. In the present embodiment, the proximal end portion 7a comes into contact with the positioning step portion 16 over the entire range in the circumferential direction C1. The dense body 22 is arranged so as to be continuous with the porous body 21 having the above configuration.

The dense body 22 forms the distal end portion 7b of the porous portion 7. In other words, the distal end end portion 7b is made of a dense body. The dense body 22 is a portion in which substantially no air holes are formed, similarly to the surface of the stem body portion 6. The dense body 22 is an annular portion that extends over the entire range of the stem 4 in the circumferential direction C1. The dense body 22 is not penetrated by bone tissue of the femur 102 and is not joined to the bone tissue. The outer peripheral surface of the dense body 22 is smoothly continuous with the surface of the distal portion 6b of the stem body portion 6.

An inner peripheral surface 7c and an outer peripheral surface 7d of the porous portion 7 are both formed by the porous body 21 and the dense body 22. Note that since the majority of the porous portion 7 (the portion excluding the distal end portion 7b) is formed by the porous body 21, the inner peripheral surface 7c and the outer peripheral surface 7d are both substantially formed by the porous body 21.

The inner peripheral surface 7c of the porous portion 7 is provided as an opposing surface that opposes the mating portion 15. The inner peripheral surface 7c is mated with the mating portion 15 of the stem body portion 6. The inner peripheral surface 7c is mated with the mating portion 15 so as to constrict the mating portion 15. Also, the inner peripheral surface 7c is fixed to the mating portion 15 by the bonding layer 8 formed using a slurry.

The bonding layer 8 is a metal layer formed by heating the mating portion 15 that has been coated with a slurry and mated with the porous portion 7.

The slurry is a muddy bonding material obtained by mixing a metal powder and a liquid. The metal powder in the slurry contains at least one of a metal and an alloy. Examples of the material for this metal powder include pure Ti, a Ti alloy (e.g., Ti-6Al-4V or Ti-6Al-2Nb-1Ta), and a Co alloy (e.g., a Co-Cr alloy). In other words, the bonding layer 8 is provided as a metal layer.

The size of the metal powder particles in the slurry is not particularly limited, and an average particle diameter of approximately 20 to 150 µm, for example, can be used. Examples of the binder component of the slurry include a starch, a sugar such as agarose, and an alcohol such as polyvinyl alcohol (PVA). The content percentage of the metal powder in the slurry is approximately 75 to 95 wt%, for example. Also, the content percentage of the binder component in the slurry is approximately 1 wt%, for example. The bonding layer 8 is formed over the entire range of the mating portion 15, and is formed over the entire range of the positioning step portion 16.

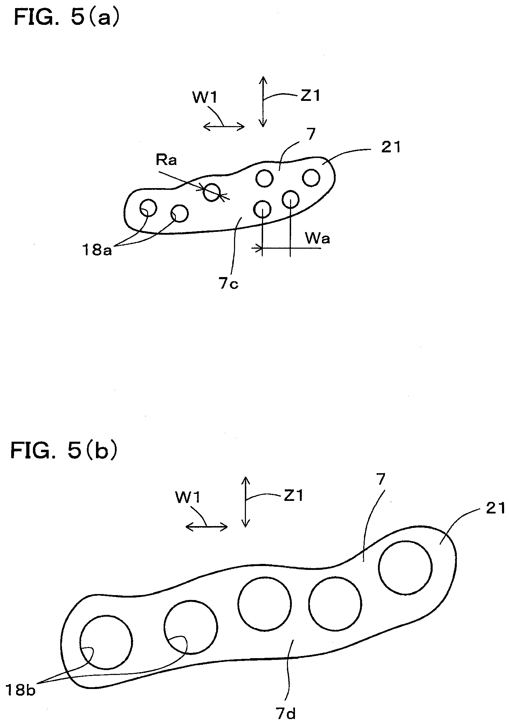

FIG. 5(a) is an enlarged schematic side view of a portion of the inner peripheral surface 7c of the porous portion 7. FIG. 5(b) is an enlarged schematic side view of a portion of the outer peripheral surface 7d of the porous portion 7. The position in the lengthwise direction Z1 is the same location in FIGS. 5(a) and 5(b).

As shown in FIGS. 3, 4, 5(a), and 5(b), many holes (openings) 18a are formed in the inner peripheral surface 7c of the porous portion 7. Also, many holes (openings) 18b are formed in the outer peripheral surface 7d of the porous portion 7. The holes 18a and 18b constitute a portion of the air holes 25. The holes 18a are configured so as to be able to allow scattering of the binder component in the slurry when binding the porous portion 7 and the stem body portion 6. Also, the holes 18b are configured so as to be able to allow scattering of the binder component, and are configured so as to be able to be penetrated by bone tissue in the proximal portion 102a of the femur 102.

A total area ratio Pa of the many holes 18a in the inner peripheral surface 7c is lower than a total area ratio Pb of the many holes 18b in the outer peripheral surface 7d (Pa<Pb). The area ratio Pa of the holes 18a refers to the ratio of the total area of all of the holes 18a to the area of the inner peripheral surface 7c of the porous portion 7. Also, the area ratio Pb of the holes 18b refers to the ratio of the total area of all of the holes 18b to the area of the outer peripheral surface 7d of the porous portion 7.

At least some of the holes 18a among the many holes 18a are in communication with one or more of the holes 18b, and thus are in communication with a space S1 on the outer peripheral surface 7d side of the porous portion 7. In the case of this configuration, when heat processing is performed to bind the porous portion 7 and the stem body portion 6 using the slurry, the binder component in the slurry can be scattered as a gas through the holes 18a and the holes 18b among the air holes 25 to the space on the outer peripheral surface 7d side of the porous portion 7. Accordingly, it is possible to suppress the formation of cavities in the portion where the porous portion 7 and the stem body portion 6 are joined, thus making it possible to ensure a greater area for the portion where the porous portion 7 and the stem body portion 6 are joined.

Also, by setting the area ratio Pb higher than the area ratio Pa, it is possible to ensure a sufficient area in which the porous portion 7 and the stem body portion 6 are joined to each other. Accordingly, through a configuration in which the holes 18a and the holes 18b are in communication with each other, and by setting the area ratio Pb higher than the area ratio Pa, it is possible to ensure sufficient joining strength between the porous portion 7 and the stem body portion 6.

The area ratio Pa of the holes 18a is preferably 30% or less, and more preferably 0.4 to 30%. By setting the area ratio Pa to 0.4% or more, the binder component in the slurry can be efficiently allowed to scatter to the space S1 outside the stem body portion 6 when heating the slurry. Also, by setting the area ratio Pa of the holes 18a to 30% or less, it is possible to ensure sufficient joining area between the porous portion 7 and the stem body portion 6, thus enabling ensuring sufficient joining strength between them. The lower limit of the area ratio Pa of the holes 18a is more preferably 0.5%, further preferably 1%, and particularly preferably 1.5%. Also, the upper limit of the area ratio Pa of the holes 18a is more preferably 27%, further preferably 25%, and particularly preferably 20%.

If the pore diameter Ra of the holes 18a is too large, there is a possibility of the slurry leaking through the holes 18a to the holes 18b and obstructing the holes 18b. If the holes 18b are obstructed, bone tissue of the femur 102 cannot penetrate into the holes 18b, and it is difficult to ensure sufficient joining strength between the proximal portion 102a of the femur 102 and the porous portion 7.

This leakage of the slurry can be avoided by adjusting the viscosity of the slurry according to the pore diameter Ra of the holes 18a, and it is preferable that the pore diameter Ra is 600 µm or less. If the pore diameter Ra is set in this way, the leakage of the slurry into the holes 18b is suppressed, and obstruction of the holes 18b by the slurry is suppressed. The pore diameter Ra is preferably 500 µm or less, and more preferably 400 µm or less. From the viewpoint of suppressing the leakage of the slurry, the smaller the pore diameter Ra is, the more preferable. The pore diameter Ra is set to 100 µm, for example.

There are no particular limitations on the shape of the holes 18a. If the holes 18a are circular, for example, the pore diameter Ra means the diameter of the circular holes 18a, and if the holes 18a are a shape other than circular, the pore diameter Ra means the equivalent circle diameter (the diameter of a circle having the same area as the area of the holes 18a). Note that although FIGS. 5(a) and 5(b) show an example of a mode in which the holes 18a and 18b are both circular, they may be a shape other than circular.

Also, it is preferable that the holes 18a are arranged uniformly in the inner peripheral surface 7c. Also, it is preferable that the shapes of the openings 18a are set to shapes that are approximately the same as each other. Also, it is preferable that a distance Wa between adjacent holes 18a in the inner peripheral surface 7c is set to 0.5 to 7.0 mm. Setting the distance Wa in this way enables efficiently allowing scattering of the binder component in the slurry to the space S1 during heating. Note that the distance Wa means the distance between the centers of adjacent holes 18a. The lower limit of the distance Wa is more preferably 1.0 mm, further preferably 1.3 mm, and particularly preferably 1.5 mm. The upper limit of the distance Wa is more preferably 6.5 mm, and further preferably 6.0 mm.

It is preferable that the length of the holes 18a in the thickness direction of the porous portion 7 is as small as possible from the viewpoint of ensuring a sufficient size of the holes 18b in order to exhibit biocompatibility. The range of the length of the holes 18a is preferably 0.5 mm or less, more preferably 0.4 mm or less, and further preferably 0.3 mm or less. Although there are no particular limitations on the lower limit of the length of the holes 18a, it is approximately 0.1 mm, for example. Note that although the holes 18a and the holes 18b closest to those holes 18a are connected to each other in the present embodiment, this need not be the case. Holes 18a and 18b that are distant from each other may be connected.

In particular, it is preferable that the above-described conditions on the area ratio Pa of the holes 18a, the pore diameter Ra, and the distance Wa between the holes 18a are all satisfied. This makes it possible to allow scattering of the binder component in the slurry from the holes 18a to the space S1 when the slurry is heated. Accordingly, it is possible to suppress the formation of cavities in the joining portion between the porous portion 7 and the stem body portion 6, and it is possible to ensure a sufficient joining area between the porous portion 7 and the stem body portion 6. It is therefore possible to improve the joining strength between the porous portion 7 and the stem body portion 6. Also, since obstruction of the holes 18b by the slurry can be suppressed, it is possible to reliably allow bone tissue of the femur 102 to penetrate into the holes 18b, and it is possible to further raise the joining strength between the proximal portion 102a of the femur 102 and the porous portion 7.

The holes 18b in the outer peripheral surface 7d are provided from the viewpoint of ensuring biocompatibility, and the area ratio Pb of the holes 18b is set large to a certain extent. This enables allowing sufficient growth of bone tissue of the femur 102 in the hole 18b, as a result making it possible to ensure sufficient bonding force between the porous portion 7 and the proximal portion 102a of the femur 102.

There are no particular limitations on the area ratio Pb of the holes 18b, and it can be set as necessary with consideration given to biocompatibility. The area ratio Pb is set to approximately 50 to 85%, for example. Also, although there are no particular limitations on the porosity of the portion of the porous body 21 of the porous portion 7 other than the inner peripheral surface 7c, it is set to approximately 50 to 85%, for example.

### Stem manufacturing steps

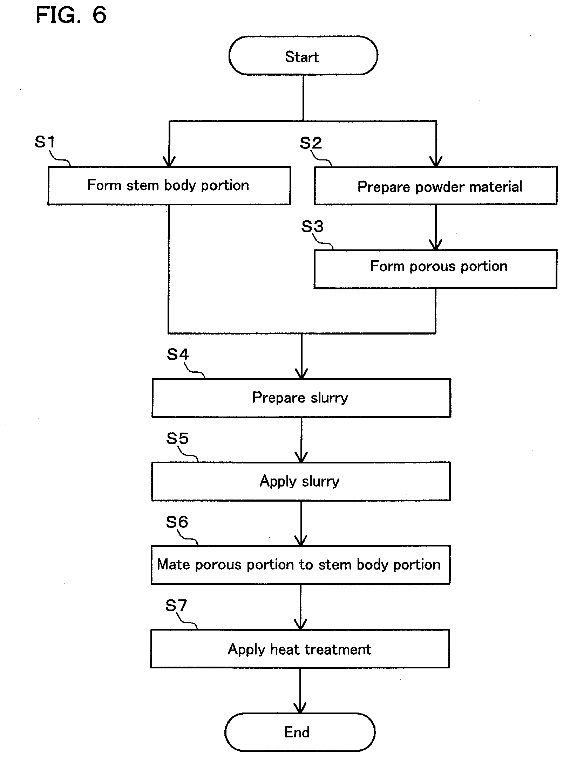

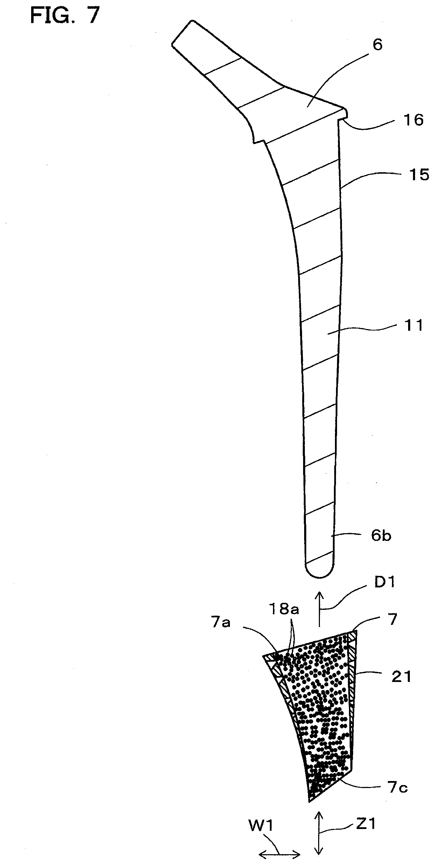

FIG. 6 is a flowchart for describing stem manufacturing steps. The stem 4 is manufactured according to the flow shown in FIG. 6. Specifically, first the stem body portion 6 shown in FIG. 7 is formed (step S1). Also, in parallel with the formation of the stem body portion 6, a metal powder material is provided for forming the porous portion 7 using a stacking and shaping method (step S2). The substance making up this powder material has already been described above. The powder material to be used in stacking and shaping can be prepared by an atomization method (a water atomization method or a gas atomization method), a rotating electrode method, a ball mill method, or the like. It is preferable that the powder prepared by any of the above-described methods is subjected to sieving or the like as necessary. This makes it possible to obtain a metal powder having an average particle diameter of 20 to 150 µm. Note that the average particle diameter of the metal powder is preferably approximately 30 to 60 µm.

Next, the porous portion 7 shown in FIG. 7 is formed using the above-described powder material (step S3). In the present embodiment, the porous portion 7 is manufactured using a stacking and shaping method. This stacking and shaping method is a method in which the same metal powder as the material for the stem 4 is stacked in layers of a predetermined thickness, and the stacked layer of metal powder is melted and allowed to solidify each time the stacking is performed so as to be shaped.

When melting the metal powder, an electromagnetic radiation beam such as a laser beam or a particle radiation beam such as an electron beam is emitted in accordance with three-dimensional image data specifying the shape of the porous portion 7. The metal powder is melted and allowed to solidify using this shaping method, thus making it possible to obtain the porous portion 7. The size (diameter) of the cross-section orthogonal to the lengthwise direction Z1 of the respective portions of the inner peripheral surface 7c of the porous portion 7 in the standalone state is the same as or larger than the size (diameter) of the cross-section of the corresponding portion of the mating portion 15 of the stem body portion 6.

The conditions in the stacking and shaping are appropriately set according to the type of powder material to be used and the shape of the porous portion 7 (the porosity of the porous body 21 of the porous portion 7, the diameter Ra of the holes 18a, the distance Wa between holes 18a, and the like). For example, the diameter of the cross-section (circle) of the radiation beam that irradiates the powder material can be set to approximately 50 to 200 µm, the distance between the radiation beam source and the powder can be set to approximately 40 to 80 cm, and the thickness of one powder layer can be set to approximately 20 to 200 µm. Also, there are no particular limitations on the atmosphere during the stacking and shaping, but it is preferable that the stacking and shaping is performed in a vacuum environment. Also, the atmosphere may be an inert gas atmosphere made of argon gas, nitrogen gas, or the like.

After the stem body portion 6 and the porous portion 7 have been completed, the slurry is prepared (step S4). The material making up this slurry has already been described above. Next, the mating portion 15 of the stem body portion 6 is coated with the slurry (step S5). Examples of methods for coating the mating portion 15 with the slurry include brush coating, roller coating, spray coating, and dip coating. Alternatively, the inner peripheral surface 7c of the porous portion 7 may be coated with the slurry.

Next, the porous portion 7 is mated with the mating portion 15 by moving the porous portion 7 toward the mating portion 15 along an arrow D1 in FIG. 7 (step S6). At this time, the porous portion 7 is inserted into the mating portion 15 of the stem body portion 6 from the distal portion 6b side of the stem body portion 6. The inner peripheral surface 7c of the porous portion 7 is then mated with the mating portion 15 so as to constrict the mating portion 15. Also, the proximal end portion 7a of the porous portion 7 comes into contact with the positioning step portion 16 of the stem body portion 6.

Next, heating for bonding the porous portion 7 and the stem body portion 6 is performed (step S7). The heating temperature and time here are appropriately set with consideration given to conditions such as the type of metal powder contained in the slurry, the extent of sintering, and the ability to sufficiently vaporize the binder component in the slurry. In the case of using a titanium-based metal, for example, the heating temperature is set to 800 to 1100°C, or preferably is set to approximately 900 to 1000°C. Also, the heating time is set to approximately 1 to 5 hours, or preferably is set to approximately 2 to 4 hours. The binder component in the slurry scatters due to this heating, thus resulting in the formation of the bonding layer 8.

As described above, according to the stem 4, the porous portion 7 is provided as a tubular standalone portion. For this reason, the stem body portion 6 can be given a simple shape for bonding with the porous portion 7. Accordingly, the stem body portion 6 has few locations that need to be processed for bonding with the porous portion 7, thus making it possible to reduce the amount of labor in the processing of the stem body portion 6. In particular, in the case where the stem body portion 6 is made of a titanium alloy, the amount of labor for processing the stem body portion 6 can be significantly reduced. Also, as previously described, the stem body portion 6 can be given a simple shape for bonding with the porous portion 7. For this reason, the stem body portion 6 has few portions where stress concentration occurs due to the shape for bonding with the porous portion 7. In this way, as a result of suppressing stress concentration in the stem body portion 6, the strength of the stem 4 can be raised even higher.

Also, the porous portion 7 is provided as a standalone portion. Accordingly, the task of bonding the porous portion 7 to the stem body portion 6 can be performed in a single task. The amount of labor required for manufacturing the stem 4 can thus be reduced.

Also, the porous portion 7 is formed by stacking layers of a metal powder. According to this configuration, even if the porous portion 7 has a complex shape, the porous portion 7 can be easily manufactured by changing how the metal powder layers are stacked. For this reason, it is possible to raise the degree of freedom in the shape of the porous portion 7, thus enabling raising the degree of freedom in the shape of the stem 4.

Accordingly, it is possible to reduce the amount of labor required for manufacturing and to raise the strength through suppressing the concentration of stress, and it is possible to provide a stem 4 that has a high degree of freedom in terms of shape.

Also, according to the stem 4, the thickness Ta of the proximal portion 21a of the porous body 21 is set larger than the thickness Tb of the distal portion 21b of the porous body 21. For this reason, in the state where the stem 4 has been inserted into the medullary cavity 102b of the femur 102, the proximal portion 21a of the porous body 21 is arranged at a relatively shallow depth from the opening 102c of the medullary cavity 102b. When the distal portion (distal portion 6b) of the stem 4 is fixed to the femur 102, the proximal portion (proximal portion 6a) of the stem 4 easily becomes wobbly. Increasing the thickness of the proximal portion 21a of the porous body makes it possible for more of the bone tissue of the femur 102 to penetrate into the proximal portion 21a. Accordingly, it is possible to ensure sufficient bonding force between the femur 102 and the proximal portion 21a near the opening 102c of the medullary cavity 102b. Also, the distal portion 21b of the porous body 21 is arranged at a relatively deep depth from the opening 102c of the medullary cavity 102b. Reducing the thickness of this distal portion 21b makes it possible to suppress the case where the bone tissue of the femur 102 penetrates more than necessary into the distal portion 21b. This makes it possible to suppress stress shielding. In other words, load from the stem 4 can be transmitted in a balanced manner to the portion of the proximal portion 102a of the femur 102 that faces the porous portion 7. As a result, it is possible to suppress bone atrophy in the proximal portion 102a, thus making it possible for the state in which the stem 4 and the femur 102 are strongly bonded to be maintained over a long period.

Also, according to the stem 4, the porous body 21 is configured such that the porosity decreases from the proximal portion 21a side of the porous body 21 toward the distal portion 21b side.

According to this configuration, a larger amount of the bone tissue of the femur 102 can penetrate into the proximal portion 21a side of the porous body 21. Accordingly, it is possible to ensure sufficient bonding force between the femur 102 and the proximal portion 21a near the opening 102c of the femur 102. Also, the distal portion 21b of the porous body 21 is arranged at a relatively deep depth from the opening 102c of the medullary cavity 102b. Reducing the porosity of the distal portion 21b makes it possible to suppress the case where the bone tissue of the femur 102 penetrates more than necessary into the distal portion 21b. This makes it possible to more reliably suppress stress shielding.

Also, according to the stem 4, the distal end portion 7b of the porous portion 7 is formed by a dense body similar to the stem body portion 6. Since the bone tissue of the femur 102 cannot penetrate into the dense body, the distal end portion 7b and the femur 102 do not become bonded to each other. Accordingly, load is more easily transmitted in the proximal portion 21a, and stress shielding can be further suppressed.

Also, according to the stem 4, the porous portion 7 is formed such that metal powder layers are successively stacked and melted temporary so as to form an integral body. According to this configuration, the porosity of the porous body 21 can be easily adjusted by changing the degree of melting metal powder, thus making it possible to further raise the degree of freedom in manufacturing the porous portion 7.

Also, according to the stem 4, the stem body portion 6 and the porous portion 7 are formed using members that are separate from each other, and are integrally bonded to each other. According to this configuration, the stem body portion 6 and the porous portion 7 can be manufactured separately. For example, the stem body portion 6 can be formed by casting, and the porous portion 7 can be formed by melting a metal powder. Accordingly, the degree of freedom in manufacturing the stem 4 can be increased. Also, the holes 18a and 18b in the porous body 21 can be manufactured more uniformly than in the case where the stem body portion 6 and the porous portion 7 are formed together.

Also, according to the stem 4, the stem body portion 6 and the porous portion 7 are bonded to each other by bonding using a slurry. In this way, the stem body portion 6 and the porous portion 7 can be bonded to each other using a simple method.

Also, according to the stem 4, multiple holes 18a are formed in the inner peripheral surface 7c of the porous portion 7. According to this configuration, the binder component in the slurry can be allowed to escape through the holes 18a when bonding the stem body portion 6 and the porous portion 7 by heating. Accordingly, it is possible to suppress the formation of cavities due to the binder component gas, and it is possible to suppress variation in the bonding force between the stem body portion 6 and the porous portion 7.

Also, according to the stem 4, the diameter of each of the holes 18a is 600 µm or less. Accordingly, it is possible to suppress the case where the liquid component of the slurry leaks into and obstructs the holes 18b in the porous portion 7, thus making it possible to ensure sufficient bonding force between the femur 102 and the porous portion 7. Also, the total area ratio of the holes 18a in the inner peripheral surface 7c is 30% or less, and the distance between adjacent holes 18a is 0.5 to 7.0 mm. Accordingly, it is possible to efficiently discharge the binder component in the slurry to the outside of the stem 4, while ensuring sufficient bonding force between the stem body portion 6 and the porous portion 7.

Also, according to the stem 4, the positioning step portion 16 is formed in the proximal portion 11a of the insertion portion 11 of the stem body portion 6. Accordingly, it is possible for the position of the porous portion 7 relative to the position of the stem body portion 6 to be more accurately defined by the positioning step portion 16.

Also, when manufacturing the stem 4, the porous portion 7 is bonded to the insertion portion 11 so as to constrict the proximal portion 11a of the insertion portion 11 of the stem body portion 6. In other words, the porous portion 7 and the insertion portion 11 can be bonded so as to form an interference fit. This makes it possible to further increase the bonding force between the porous portion 7 and the insertion portion 11. It is also possible to suppress the case where the porous portion 7 becomes separated from the insertion portion 11 during the task of bonding the porous portion 7 and the insertion portion 11.

Incidentally, with the configuration disclosed in previously-described Patent Document 1, it is conceivable for the edge portions of the porous bodies to protrude from the recessions in the stem body due to the dimensional tolerance of the stem body and the dimensional tolerance of the plate-shaped porous bodies. If the edge portions of the porous bodies protrude from the recessions, there are cases where, when the stem is inserted into the medullary cavity of the femur, only the portions of the porous bodies on the stem distal portion side come into contact with and become caught on the inner surface of the hole portion of the femur. In other words, there is the possibility that only the portion of the porous bodies, which have a large surface area, on the distal portion side will bond with the bone tissue of the femur in an abutted edge manner. In the case of this type of bonding, stress from the femoral stem will not act on portions of the femur that should be bonded with the porous bodies, and thus stress shielding occurs in the femur. Accordingly, it is not possible to maintain a state of stable bonding between the femoral stem and the femur, and the femoral stem wobbles relative to the femur. If this wobbling occurs, it is necessary to perform an operation (reoperation) for firmly attaching the femoral stem to the proximal portion of the femur, which is troublesome.

Furthermore, with the configuration disclosed in Patent Document 1, when the femoral stem is inserted into the medullary cavity of the femur, there is the possibility that the portions of the porous bodies that protrude from the recession will become caught inside the femur, and it will not be possible to insert the femoral stem to the desired depth. In this case, similarly to the above-described case, load from the femoral stem will not act on portions of the femur that should be bonded with the porous bodies.

Furthermore, with the configuration disclosed in Patent Document 1, when the femoral stem is inserted into the medullary cavity of the femur, there is the possibility that the porous bodies will become caught on the inner wall portion of the femur, and the porous bodies will become detached from the stem body. If the porous bodies have been provided with an apatite coating, there is also the possibility of the coating layer becoming detached.

In contrast, according to the stem 4, the outer peripheral surface of the stem body portion 6 and the outer peripheral surface 7d of the porous portion 7 are connected so as to be smoothly continuous, and it is possible to suppress the case where the porous portion 7 becomes caught on the femur 102 in the medullary cavity 102b. This makes it possible to ensure sufficient contact area between the porous portion 7 and the femur 102. Accordingly, the portions of the femur 102 that should be bonded with the porous portion 7 can be reliably brought into contact with the porous portion 7. This makes it possible to suppress stress shielding as described above. Also, since it is possible to suppress the case where the stem 4 becomes caught on the medullary cavity 102b of the femur 102, the stem 4 can be reliably inserted to the desired depth in the medullary cavity 102b, and chipping of the outer peripheral surface 7d of the porous portion 7 can be suppressed.

Although an embodiment of the present invention has been described thus far, the present invention is not limited to the above-described embodiment, and various modifications can be made within the scope recited in the claims. For example, the following modifications are possible.

### Variations

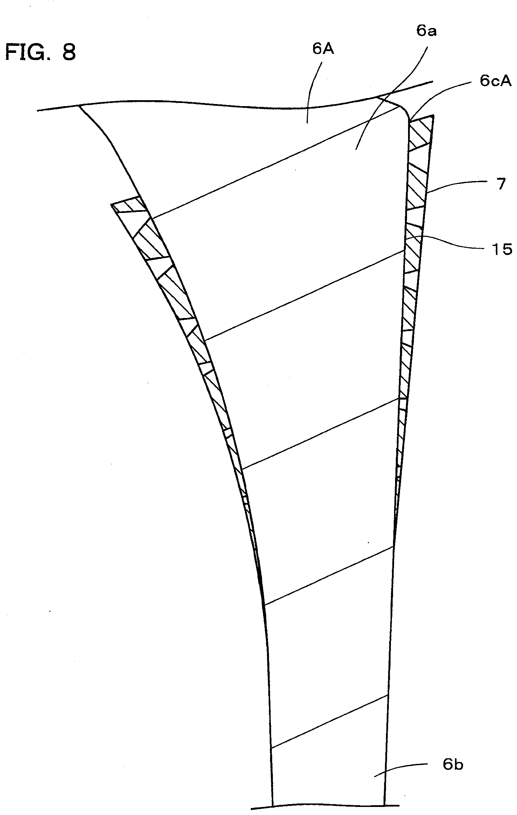

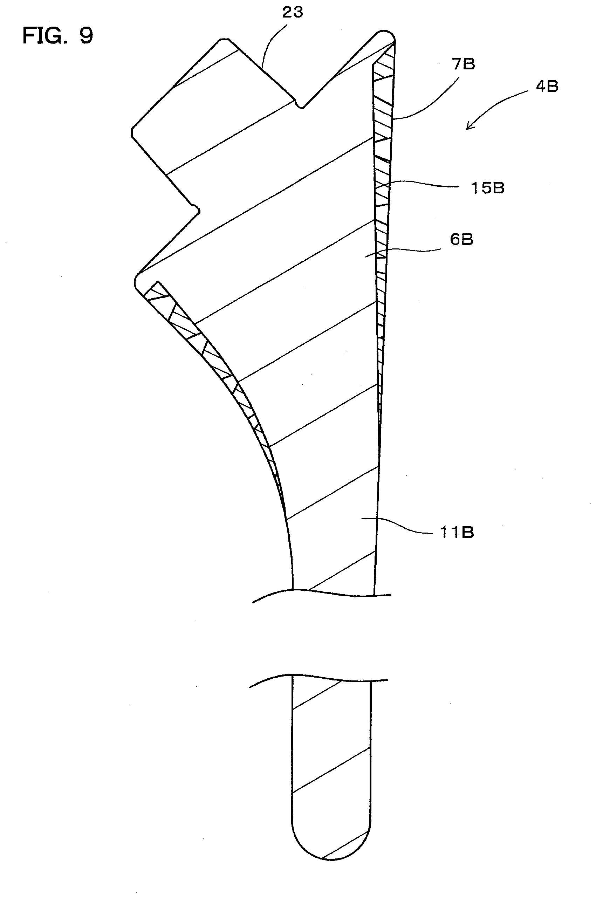

(1) Although the above-described embodiment describes an example of a mode in which the porous portion 7 and the stem body portion 6 are bonded using a slurry, this need not be the case. For example, the porous portion 7 and the stem body portion 6 may be bonded without using a slurry. More specifically, the porous portion 7 and the stem body portion 6 may be bonded using diffusion joining. Diffusion joining (solid layer diffusion joining) refers to joining in which the porous portion 7 and the stem body portion 6 are brought into close contact and pressed together so as to become joined using atomic diffusion that occurs between the opposing surfaces of the porous portion 7 and the stem body portion 6.
   In the case of performing diffusion joining, in the above-described embodiment, the inner peripheral surface 7c of the porous portion 7 is formed as a dense body. Also, the step for preparing the slurry (step S4) is omitted. Also, in the above-described heating step (step S7), heating is performed with a temperature of 900 to 1100°C in the case of using a titanium-based metal, for example. At this time, the porous portion 7 is pressed against the stem body portion 6. One example of the pressure application method here is hot isostatic pressing (HIP). The porous portion 7 and the stem body portion 6 can be easily bonded using diffusion joining.
(2) Also, although the above-described embodiment describes an example of a mode in which the positioning step portion 16 is provided on the stem body portion 6, this need not be the case. For example, the positioning step portion 16 does not need to be formed on a proximal end portion 6cA of a stem body portion 6A as shown in FIG. 8. Note that the following describes a different configuration from the above configuration, and portions of the configuration similar to the above configuration will be denoted by the same reference signs in the drawings and will not be described.
   In this case, a step is not formed on the stem body portion 6A, and the surface is smooth and continuous from the proximal portion 6a of the stem body portion 6A to the distal portion 6b. In other words, the outer peripheral portion of the stem body portion 6A is formed by a smooth surface. In this case, there is no need for processing to form a positioning step portion on the stem body 6A. This makes it possible to reduce the amount of labor in the processing of the stem body portion 6A. Also, the stem body portion 6A can be given a simple shape for bonding with the porous portion 7. For this reason, the stem body portion 6A has even fewer portions where stress concentration occurs due to the shape for bonding with the porous portion 7. In this way, as a result of suppressing stress concentration in the stem body portion 6A, the strength of the stem can be raised even higher.
(3) Also, although the above-described embodiment describes an example of a mode in which the porous portion is formed using a stacking and shaping method, this need not be the case. For example, the porous portion may be formed using sintering.
(4) Also, although the above-described embodiment describes an example of a mode in which the porous portion has an endless tube shape, this need not be the case. For example, a slit may be formed at a location in the circumferential direction of the porous portion. This slit passes through the porous portion in the lengthwise direction, and the porous portion is formed in the shape of an approximately C-shaped tube. In this case, the porous portion can be more easily brought into close contact with the mating portion of the stem body portion 6 with the application of pressure, thus making it possible to more easily raise the bonding force between the porous portion and the stem body portion.
(5) Also, although the above-described embodiment describes an example of a mode in which one porous portion is bonded to the stem body portion, this need not be the case. For example, multiple porous portions may be bonded to the stem body portion. In this case, the porous portions are aligned along the lengthwise direction.
(6) Although the above-described embodiment describes an example of a mode in which the thickness and the porosity of the porous body both change in a continuous manner, this need not be the case. For example, the modes of the thickness and the porosity of the porous body can both be modes different from the above embodiment.
(7) Also, although the above-described embodiment describes an example of a stem used in an artificial hip joint as an artificial joint stem, this need not be the case. For example, the artificial joint component of the present invention may be applied to at least one of an artificial elbow joint and an artificial shoulder joint. FIG. 9 is a cross-sectional diagram showing a stem 4B as an artificial joint stem used in an artificial shoulder joint. The stem 4B is configured so as to be inserted into the humerus (not shown) of a biological body. An insertion portion 11B of a stem body portion 6B of the stem 4B is provided with an integrated protrusion 23 for fixing a head (not shown) shaped as an approximately semi-elliptical sphere. A porous portion 7B is bonded to a mating portion 15B of the stem 4B.

### Industrial Applicability

The present invention is broadly applicable as an artificial joint stem used in an operation for replacing a patient's joint with an artificial joint, as well as an artificial joint stem component and an artificial joint stem manufacturing method.

### Descriptions of Reference Numerals

- 4: Stem (artificial joint stem)
- 6: Stem body portion
- 7: Porous portion
- 11: Insertion portion
- 11a: Proximal portion of insertion portion
- 21: Porous body
- 102: Femur (bone)
- 102b: Medullary cavity

## Claims

1. An artificial joint stem (4;4B), comprising:
(a) a stem body portion (6;6A;6B); and
(b) a porous portion (7;7B) that has a porous body (21) and is bonded to the stem body portion (6;6A;6B),
(c) wherein the stem body portion (6;6A;6B) has an insertion portion (11;11B) arranged to be inserted into a medullary cavity (102b) of a bone (102) of a biological body, and
(d) the porous portion (7;7B) is a tubular standalone portion formed by stacking layers of a biocompatible metal powder, and is arranged so as to surround a proximal portion (11a) of the insertion portion (11;11B); **characterized in that**
(e) the thickness (Ta) of a proximal portion (21a) of the porous body (21) is set larger than the thickness (Tb) of a distal portion (21b) of the porous body (21);
(f1) wherein a multitude of inner openings (18a) are formed in the inner peripheral surface (7c) of the porous portion (7; 7B) and a multitude of outer openings (18b) are formed in the outer peripheral surface (7d) of the porous portion (7;7A); and
(f2) wherein a total area ratio (Pa) of the inner openings (18a) in the inner peripheral surface (7c) is lower than the total area ratio (Pb) of the outer openings (18b) in the outer peripheral surface (7d), wherein the total area ratio (Pa) of the inner openings (18a) refer to the ratio of the total area of all the inner openings (18a) to the area of the inner peripheral surface (7c) of the porous portion (7B) and wherein the total area ratio (Pb) of the outer openings (18b) refer to the ratio of the total area of all the outer openings (18b) to the area of the outer peripheral surface (7d) of the porous portion (7;7B).

2. The artificial joint stem (4;4B) according to claim 1,
wherein at least some of the inner openings (18a) are in communication with one or more of the outer openings (18b).

3. The artificial joint stem (4;4B) according to one of claims 1 and 2,
wherein the porous body (21) is configured such that the porosity of the porous body (21) decreases from the proximal portion (21a) side of the porous body (21) toward the distal portion (21b) side of the porous body (21).

4. The artificial joint stem (4;4B) according to any one of claims 1 to 3,
wherein a distal end portion (7b) of the porous portion (7;7B) is a dense body.

5. The artificial joint stem (4;4B) according to any one of claims 1 to 4,
wherein the porous portion (7;7B) is formed in an integrated state by successively stacking and temporary melting layers of the metal powder.

6. The artificial joint stem (4;4B) according to any one of claims 1 to 5,
wherein the stem body portion (6;6A;6B) and the porous portion (7;7B) are formed using members that are separate from each other, and are integrally bonded to each other.

7. The artificial joint stem (4;4B) according to claim 6,
wherein the stem body portion (6;6A;6B) and the porous portion (7;7B) are bonded to each other by bonding using a slurry or bonding using diffusion joining.

8. The artificial joint stem (4;4B) according to claim 7,
wherein the stem body portion (6;6A;6B) and the porous portion (7;7B) are bonded to each other by bonding using the slurry,
the porous portion (7;7B) has an opposing surface (7c) that opposes the proximal portion (11a) of the insertion portion (11;11B), and
a plurality of opening portions (18a) are formed in the opposing surface (7c).

9. The artificial joint stem (4;4B) according to claim 8,
wherein at least one condition is satisfied among conditions that the diameter (Ra) of each of the opening portions (18a) is 600 µm or less, the total area ratio (Pa) of the plurality of opening portions (18a) in the opposing surface (7c) is 30% or less, and the distance (Wa) between adjacent opening portions (18a) is 0.5 to 7.0 mm.

10. The artificial joint stem (4;4B) according to any one of claims 1 to 9,
wherein a positioning step portion (16) for defining the position of the porous portion (7;7B) relative to the stem body portion (6;6A;6B) is formed in a proximal portion (11a) of the insertion portion (11;11B).

11. An artificial joint stem component (7;7B) that is a component to be bonded to a stem body portion (6;6A;6B) to be inserted into a medullary cavity (102b) of a bone (102) of a biological body, the artificial joint stem component (7;7B) comprising:
(a) a porous portion (7;7B) that has a porous body (21) and is to be bonded to the stem body portion (6;6A;6B),
(b) wherein the porous portion (7;7B) is a tubular standalone component formed by stacking layers of a biocompatible metal powder; **characterized in that**
(c) the thickness (Ta) of a proximal portion (21a) of the porous body (21) is set larger than the thickness (Tb) of a distal portion (21b) of the porous body (21);
(d1) wherein a multitude of inner openings (18a) are formed in the inner peripheral surface (7c) of the porous portion (7; 7B) and a multitude of outer openings (18b) are formed in the outer peripheral surface (7d) of the porous portion (7;7A); and
(d2) wherein a total area ratio (Pa) of the inner openings (18a) in the inner peripheral surface (7c) is lower than the total area ratio (Pb) of the outer openings (18b) in the outer peripheral surface (7d), wherein the total area ratio (Pa) of the inner openings (18a) refer to the ratio of the total area of all the inner openings (18a) to the area of the inner peripheral surface (7c) of the porous portion (7B) and wherein the total area ratio (Pb) of the outer openings (18b) refer to the ratio of the total area of all the outer openings (18b) to the area of the outer peripheral surface (7d) of the porous portion (7;7B).

12. A method of manufacturing an artificial joint stem (4;4B) that has a stem body portion (6;6A;6B) having an insertion portion (11;11B) that is to be inserted into a medullary cavity (102b) of a bone (102) of a biological body, and a porous portion (7;7B) that has a porous body (21) and is bonded to the stem body portion (6;6A;6B), the method comprising:
(a) a stem body portion (6;6A;6B) formation step of forming the stem body portion (6;6A;6B);
(b) a porous portion (7;7B) formation step of forming the porous portion (7;7B); and
(c) a bonding step of bonding the stem body portion (6;6A;6B) to the porous portion (7;7B),
(d) wherein in the porous portion (7;7B) formation step, the porous portion (7;7B) is formed by forming a tubular standalone component by stacking layers of a biocompatible metal powder, and
(e) in the bonding step, the porous portion (7;7B) is bonded to the stem body portion (6;6A;6B) in a state in which the porous portion (7;7B) is mated with a proximal portion (11a) of the insertion portion (11;11B); **characterized in that**
(f) the thickness (Ta) of a proximal portion (21a) of the porous body (21) is set larger than the thickness (Tb) of a distal portion (21b) of the porous body (21);
(g1) wherein a multitude of inner openings (18a) are formed in the inner peripheral surface (7c) of the porous portion (7; 7B) and a multitude of outer openings (18b) are formed in the outer peripheral surface (7d) of the porous portion (7;7A); and
(g2) wherein a total area ratio (Pa) of the inner openings (18a) in the inner peripheral surface (7c) is lower than the total area ratio (Pb) of the outer openings (18b) in the outer peripheral surface (7d), wherein the total area ratio (Pa) of the inner openings (18a) refer to the ratio of the total area of all the inner openings (18a) to the area of the inner peripheral surface (7c) of the porous portion (7B) and wherein the total area ratio (Pb) of the outer openings (18b) refer to the ratio of the total area of all the outer openings (18b) to the area of the outer peripheral surface (7d) of the porous portion (7;7B).

13. The method of manufacturing an artificial joint stem (4;4B) according to claim 12,
wherein in the bonding step, the porous portion (7;7B) is bonded to the insertion portion (11;11B) so as to constrict the proximal portion (11a) of the insertion portion (11;11B).

14. The method of manufacturing an artificial joint stem (4;4B) according to claim 12 or 13,
wherein in the bonding step, the porous portion (7;7B) and the insertion portion (11;11B) are bonded by interposing a slurry between the porous portion (7;7B) and the insertion portion (11;11B) and heating the slurry, or by performing diffusion joining on the porous portion (7;7B) and the insertion portion (11;11B).

15. The method of claim 12, wherein at least some of the inner openings (18a) are formed so as to be in communication with one or more of the outer openings (18b).

## Patentansprüche

1. Künstlicher Gelenkschaft (4; 4B), umfassend:
(a) einen Schaftkörperabschnitt (6; 6A; 6B); und
(b) einen porösen Abschnitt (7; 7B), der einen porösen Körper (21) aufweist und mit dem Schaftkörperabschnitt (6; 6A; 6B) verbunden ist,
(c) wobei der Schaftkörperabschnitt (6; 6A; 6B) einen Einführungsabschnitt (11; 11B) aufweist, der ausgebildet ist, in einen Markraum (102b) eines Knochens (102) eines biologischen Körpers eingeführt zu werden, und
(d) wobei der poröse Abschnitt (7; 7B) ein rohrförmiger selbständiger Abschnitt ist, gebildet durch Stapeln von Schichten eines biokompatiblen Metallpulvers, und derart ausgebildet ist, dass er einen proximalen Abschnitt (11a) des Einführungsabschnitts (11; 11B) umgibt; **dadurch gekennzeichnet, dass**
(e) die Dicke (Ta) eines proximalen Abschnitts (21a) des porösen Körpers (21) größer als die Dicke (Tb) eines distalen Abschnitts (21b) des porösen Körpers (21) gestaltet ist;
(f1) wobei eine Vielzahl von inneren Öffnungen (18a) in der inneren Umfangsfläche (7c) des porösen Abschnitts (7; 7B) ausgebildet sind und eine Vielzahl von äußeren Öffnungen (18b) in der äußeren Umfangsfläche (7d) des porösen Abschnitts (7; 7A) ausgebildet sind; und
(f2) wobei ein Gesamtflächenverhältnis (Pa) der inneren Öffnungen (18a) in der inneren Umfangsfläche (7c) kleiner ist als das Gesamtflächenverhältnis (Pb) der äußeren Öffnungen (18b) in der äußeren Umfangsfläche (7d), wobei das Gesamtflächenverhältnis (Pa) der inneren Öffnungen (18a) sich auf das Verhältnis der Gesamtfläche sämtlicher inneren Öffnungen (18a) zu der Fläche der inneren Umfangsfläche (7c) des porösen Abschnitts (7B) bezieht, und wobei das Gesamtflächenverhältnis (Pb) der äußeren Öffnungen (18b) sich auf das Verhältnis der Gesamtfläche sämtlicher äußeren Öffnungen (18b) zu der Fläche der äußeren Umfangsfläche (7d) des porösen Abschnitts (7; 7B) bezieht.

2. Künstlicher Gelenkschaft (4; 4B) nach Anspruch 1,
wobei mindestens einige der inneren Öffnungen (18a) mit einer oder mehreren der äußeren Öffnungen (18b) in Verbindung stehen.

3. Künstlicher Gelenkschaft (4; 4B) nach einem der Ansprüche 1 und 2,
wobei der poröse Körper (21) derart ausgebildet ist, dass die Porosität des porösen Körpers (21) von der Seite des proximalen Abschnitts (21a) des porösen Körpers (21) zu der Seite des distalen Abschnitt (21b) des porösen Körpers (21) abnimmt.

4. Künstlicher Gelenkschaft (4; 4B) nach einem der Ansprüche 1 bis 3,
wobei ein distaler Endabschnitt (7b) des porösen Abschnitts (7; 7B) ein dichter Körper ist.

5. Künstlicher Gelenkschaft (4; 4B) nach einem der Ansprüche 1 bis 4,
wobei der poröse Abschnitt (7; 7B) in einem integrierten Zustand durch aufeinanderfolgendes Stapeln und temporäres Schmelzen von Schichten des Metallpulvers gebildet ist.

6. Künstlicher Gelenkschaft (4; 4B) nach einem der Ansprüche 1 bis 5,
wobei der Schaftkörperabschnitt (6; 6A; 6B) und der poröse Abschnitt (7; 7B) unter Verwendung von Elementen, die voneinander getrennt sind, gebildet und integral miteinander gebildet sind.

7. Künstlicher Gelenkschaft (4; 4B) nach Anspruch 6,
wobei der Schaftkörperabschnitt (6; 6A; 6B) und der poröse Abschnitt (7; 7B) durch Bindung unter Verwendung einer Aufschlämmung oder durch Bindung unter Verwendung von Diffusionsverbinden miteinander verbunden sind.

8. Künstlicher Gelenkschaft (4; 4B) nach Anspruch 7,
wobei der Schaftkörperabschnitt (6; 6A; 6B) und der poröse Abschnitt (7; 7B) durch Bindung unter Verwendung der Aufschlämmung miteinander verbunden sind,
wobei der poröse Abschnitt (7; 7B) eine gegenüberliegende Fläche (7c) aufweist, die dem proximalen Abschnitt (11a) des Einführungsabschnitts (11; 11B) gegenüberliegt, und wobei eine Mehrzahl von Öffnungsabschnitten (18a) in der gegenüberliegenden Fläche (7c) ausgebildet sind.

9. Künstlicher Gelenkschaft (4; 4B) nach Anspruch 8,
wobei mindestens eine Bedingung von den Bedingungen, dass der Durchmesser (Ra) jedes der Öffnungsabschnitte (18a) 600 µm oder weniger ist, dass das Gesamtflächenverhältnis (Pa) der Mehrzahl von Öffnungsabschnitten (18a) in der gegenüberliegenden Fläche (7c) 30 % oder weniger ist, und dass der Abstand (Wa) zwischen benachbarten Öffnungsabschnitten (18a) 0,5 bis 7,0 mm ist, erfüllt ist.

10. Künstlicher Gelenkschaft (4; 4B) nach einem der Ansprüche 1 bis 9,
wobei ein Positionier-Stufenabschnitt (16) zum Definieren der Position des porösen Abschnitts (7; 7B) relativ zu dem Schaftkörperabschnitt (6; 6A; 6B) in einem proximalen Abschnitt (11a) des Einführungsabschnitts (11; 11B) gebildet ist.

11. Künstliche Gelenkschaftkomponente (7; 7B), die eine Komponente ist, die mit einem Schaftkörperabschnitt (6; 6A; 6B) zum Einführen in einen Markraum (102b) eines Knochens (102) eines biologischen Körpers verbunden ist, wobei die künstliche Gelenkschaftkomponente (7; 7B) Folgendes umfasst:
(a) einen porösen Abschnitt (7; 7B), der einen porösen Körper (21) aufweist und mit dem Schaftkörperabschnitt (6; 6A; 6B) zu verbinden ist,
(b) wobei der poröse Abschnitt (7; 7B) eine durch Stapeln von Schichten eines biokompatiblen Metallpulvers gebildete rohrförmige selbständige Komponente ist; **dadurch gekennzeichnet, dass**
(c) die Dicke (Ta) eines proximalen Abschnitts (21a) des porösen Körpers (21) größer als die Dicke (Tb) eines distalen Abschnitts (21b) des porösen Körpers (21) gestaltet ist;
(d1) wobei eine Vielzahl von inneren Öffnungen (18a) in der inneren Umfangsfläche (7c) des porösen Abschnitts (7; 7B) ausgebildet sind und eine Vielzahl von äußeren Öffnungen (18b) in der äußeren Umfangsfläche (7d) des porösen Abschnitts (7; 7A) ausgebildet sind; und
(d2) wobei ein Gesamtflächenverhältnis (Pa) der inneren Öffnungen (18a) in der inneren Umfangsfläche (7c) kleiner ist als das Gesamtflächenverhältnis (Pb) der äußeren Öffnungen (18b) in der äußeren Umfangsfläche (7d), wobei das Gesamtflächenverhältnis (Pa) der inneren Öffnungen (18a) sich auf das Verhältnis der Gesamtfläche sämtlicher inneren Öffnungen (18a) zu der Fläche der inneren Umfangsfläche (7c) des porösen Abschnitts (7B) bezieht und wobei das Gesamtflächenverhältnis (Pb) der äußeren Öffnungen (18b) sich auf das Verhältnis der Gesamtfläche sämtlicher äußeren Öffnungen (18b) zu der Fläche der äußeren Umfangsfläche (7d) des porösen Abschnitts (7; 7B) bezieht.

12. Verfahren zum Herstellen eines künstlichen Gelenkschafts (4; 4B), der einen Schaftkörperabschnitt (6; 6A; 6B) mit einem Einführungsabschnitt (11; 11B), der in einen Markraum (102b) eines Knochens (102) eines biologischen Körpers einzuführen ist, und einen porösen Abschnitt (7; 7B) aufweist, der einen porösen Körper (21) aufweist und mit dem Schaftkörperabschnitt (6; 6A; 6B) verbunden ist, wobei das Verfahren Folgendes umfasst:
(a) einen Bildungsschritt für den Schaftkörperabschnitt (6; 6A; 6B) zum Bilden des Schaftkörperabschnitts (6; 6A; 6B);
(b) einen Bildungsschritt für den porösen Abschnitt (7; 7B) zum Bilden des porösen Abschnitts (7; 7B); und
(c) einen Verbindungsschritt zum Verbinden des Schaftkörperabschnitts (6; 6A; 6B) mit dem porösen Abschnitt (7; 7B),
(d) wobei in dem Bildungsschritt für den porösen Abschnitt (7; 7B) der poröse Abschnitt (7; 7B) gebildet wird, indem eine rohrförmige selbständige Komponente durch Stapeln von Schichten eines biokompatiblen Metallpulvers gebildet wird, und
(e) in dem Verbindungsschritt der poröse Abschnitt (7; 7B) mit dem Schaftkörperabschnitt (6; 6A; 6B) in einem Zustand verbunden wird, in dem der poröse Abschnitt (7; 7B) mit einem proximalen Abschnitt (11a) des Einführungsabschnitts (11; 11B) zusammengefügt ist, **dadurch gekennzeichnet, dass**
(f) die Dicke (Ta) eines proximalen Abschnitts (21a) des porösen Körpers (21) größer als die Dicke (Tb) eines distalen Abschnitts (21b) des porösen Körpers (21) gestaltet wird;
(g1) wobei eine Vielzahl von inneren Öffnungen (18a) in der inneren Umfangsfläche (7c) des porösen Abschnitts (7; 7B) ausgebildet werden und eine Vielzahl von äußeren Öffnungen (18b) in der äußeren Umfangsfläche (7d) des porösen Abschnitts (7; 7A) ausgebildet werden; und
(g2) wobei ein Gesamtflächenverhältnis (Pa) der inneren Öffnungen (18a) in der inneren Umfangsfläche (7c) kleiner gestaltet wird als das Gesamtflächenverhältnis (Pb) der äußeren Öffnungen (18b) in der äußeren Umfangsfläche (7d), wobei das Gesamtflächenverhältnis (Pa) der inneren Öffnungen (18a) sich auf das Verhältnis der Gesamtfläche sämtlicher inneren Öffnungen (18a) zu der Fläche der inneren Umfangsfläche (7c) des porösen Abschnitts (7B) bezieht und wobei das Gesamtflächenverhältnis (Pb) der äußeren Öffnungen (18b) sich auf das Verhältnis der Gesamtfläche sämtlicher äußeren Öffnungen (18b) zu der Fläche der äußeren Umfangsfläche (7d) des porösen Abschnitts (7; 7B) bezieht.

13. Verfahren zur Herstellung eines künstlichen Gelenkschafts (4; 4B) nach Anspruch 12, wobei in dem Verbindungsschritt der poröse Abschnitt (7; 7B) mit dem Einführungsabschnitt (11; 11B) derart verbunden wird, dass der proximale Abschnitt (11a) des Einführungsabschnitts (11; 11B) eingeengt wird.

14. Verfahren zur Herstellung eines künstlichen Gelenkschafts (4; 4B) nach Anspruch 12 oder 13,
wobei in dem Verbindungsschritt der poröse Abschnitt (7; 7B) und der Einführungsabschnitt (11; 11B) durch Zwischenlage einer Aufschlämmung zwischen dem porösen Abschnitt (7; 7B) und dem Einführungsabschnitt (11; 11B) und Erwärmen der Aufschlämmung oder Ausführen einer Diffusionsverbindung an dem porösen Abschnitt (7; 7B) und dem Einführungsabschnitt (11; 11B) verbunden werden.

15. Verfahren nach Anspruch 12, wobei mindestens einige der inneren Öffnungen (18a) derart ausgebildet sind, dass sie mit einer oder mehreren der äußeren Öffnungen (18b) in Verbindung stehen.

## Revendications

1. Tige de joint artificiel (4;4B), comprenant :
(a) une partie de corps de tige (6;6A;6B) ; et
(b) une partie poreuse (7;7B) qui a un corps poreux (21) et est liée à la partie de corps de tige (6;6A;6B),
(c) dans laquelle la partie de corps de tige (6;6A;6B) a une partie d'insertion (11;11B) disposée pour être insérée dans une cavité médullaire (102b) d'un os (102) d'un corps biologique, et
(d) la partie poreuse (7;7B) est une partie autonome tubulaire formée en empilant des couches d'une poudre métallique biocompatible, et est disposée pour entourer une partie proximale (11a) de la partie d'insertion (11;11B) ; **caractérisée en ce que**
(e) l'épaisseur (Ta) d'une partie proximale (21a) du corps preux (21) est établie pour être plus grande que l'épaisseur (Tb) d'une partie distale (21b) du corps poreux (21) ;
(f) dans laquelle une multitude d'ouvertures internes (18a) sont formées dans la surface périphérique interne (7c) de la partie poreuse (7;7B) et une multitude d'ouvertures externes (18b) sont formées dans la surface périphérique externe (7d) de la partie poreuse (7;7A) ; et
(f2) dans laquelle un rapport de surface totale (Pa) des ouvertures internes (18a) dans la surface périphérique interne (7c) est inférieur au rapport de surface totale (Pb) des ouvertures externes (18b) dans la surface périphérique externe (7d), dans laquelle le rapport de surface totale (Pa) des ouvertures internes (18a) se rapporte au rapport de la surface totale de toutes les ouvertures internes (18a) à la surface de la surface périphérique interne (7c) de la partie poreuse (7B) et dans laquelle le rapport de surface totale (Pb) des ouvertures externes (18b) se rapporte au rapport de la surface totale de toutes les ouvertures externes (18b) à la surface de la surface périphérique externe (7d) de la partie poreuse (7;7B).

2. Tige de joint artificiel (4;4B) selon la revendication 1,
dans laquelle au moins certaines des ouvertures internes (18a) sont en communication avec une ou plusieurs des ouvertures externes (18b).

3. Tige de joint artificiel (4;4B) selon l'une des revendications 1 et 2, dans laquelle le corps poreux (21) est configuré de telle manière que la porosité du corps poreux (21) diminue à partir du côté de partie proximale (21a) du corps poreux (21) vers le côté de partie distale (21b) du corps poreux (21).

4. Tige de joint artificiel (4;4B) selon l'une quelconque des revendications 1 à 3, dans laquelle une partie d'extrémité distale (7b) de la partie poreuse (7;7B) est un corps dense.

5. Tige de joint artificiel (4;4B) selon l'une quelconque des revendications 1 à 4, dans laquelle la partie poreuse (7;7B) est formée dans un état intégré par empilement successif et fusion temporaire des couches de la poudre métallique.

6. Tige de joint artificiel (4;4B) selon l'une quelconque des revendications 1 à 5, dans laquelle la partie de corps de tige (6;6A;6B) et la partie poreuse (7;7B) sont formées en utilisant des éléments qui sont distincts l'un de l'autre, et sont liés intégralement l'un à l'autre.

7. Tige de joint artificiel (4;4B) selon la revendication 6,
dans laquelle la partie de corps de tige (6;6A;6B) et la partie poreuse (7;7B) sont liées l'une à l'autre par liaison en utilisant une suspension épaisse ou par liaison en utilisant une jonction de diffusion.

8. Tige de joint artificiel (4;4B) selon la revendication 7,
dans laquelle la partie de corps de tige (6;6A;6B) et la partie poreuse (7;7B) sont liées l'une à l'autre par liaison en utilisant la suspension épaisse,
la partie poreuse (7;7B) a une surface opposante (7c) qui oppose la partie proximale (11a) de la partie d'insertion (11;11B), et
une pluralité de parties d'ouvertures (18a) sont formées dans la surface opposante (7c).

9. Tige de joint artificiel (4;4B) selon la revendication 8,
dans laquelle au moins une condition est satisfaite parmi les conditions que le diamètre (Ra) de chacune des parties d'ouverture (18a) est de 600 µm ou moins, le rapport de surface totale (Pa) de la pluralité de parties d'ouverture (18a) dans la surface opposante (7c) est de 30 % ou moins, et la distance (Wa) entre les parties d'ouverture adjacentes (18a) est de 0,5 à 7,0 mm.

10. Tige de joint artificiel (4;4B) selon l'une quelconque des revendications 1 à 9, dans laquelle une partie d'étape de positionnement (16) pour définir la position de la partie poreuse (7;7B) par rapport à la partie de corps de tige (6;6A;6B) est formée dans une partie proximale (11a) de la partie d'insertion (11;11B).

11. Composant de tige de joint artificiel (7;7B) qui est un composant à lier à une partie de corps de tige (6;6A;6B) à insérer dans une cavité médullaire (102b) d'un os (102) d'un corps biologique, le composant de tige de joint artificiel (7;7B) comprenant :
(a) une partie poreuse (7;7b) qui a un corps poreux (21) et doit être liée à la partie du corps de tige (6; 6A; 6B),
(b) dans lequel la partie poreuse (7;7B) est un composant autonome tubulaire formé par l'empilement de couches d'une poudre métallique biocompatible ; **caractérisé en ce que**
(c) l'épaisseur (Ta) d'une partie proximale (21a) du corps poreux (21) est établie pour être plus grande que l'épaisseur Tb d'une partie distale (21b) du corps poreux (21) ;
(d1) dans lequel une multitude d'ouvertures internes (18a) sont formées dans la surface périphérique interne (7c) de la partie poreuse (7;7B) et une multitude d'ouvertures externes (18b) sont formées dans la surface périphérique externe (7d) de la partie poreuse (7;7A) ; et
(d2) dans lequel un rapport de surface totale (Pa) des ouvertures internes (18a) dans la surface périphérique interne (7c) est inférieur au rapport de surface totale (Pb) des ouvertures externes (18b) dans la surface périphérique externe (7d), dans lequel le rapport de surface totale (Pa) des ouvertures internes (18a) se rapporte au rapport de la surface totale de toutes les ouvertures internes (18a) à la surface de la surface périphérique interne (7c) de la partie poreuse (7B) et dans lequel le rapport de surface totale (Pb) des ouvertures externes (18b) se rapporte à la surface totale de toutes les ouvertures externes (18b) à la surface de la surface périphérique externe (7d) de la partie poreuse (7;7B).

12. Procédé de fabrication d'une tige de joint artificiel (4;4B) qui a une partie de corps de tige (6;6A;6B) ayant une partie d'insertion (11;11B) qui doit être insérée dans une cavité médullaire (102b) d'un os (102) d'un corps biologique, et une partie poreuse (7;7B) qui a un corps poreux (21) et est liée à la partie de corps de tige (6;6A;6B), le procédé comprenant :
(a) une étape de formation d'une partie de corps de tige (6;6A;6B) de formation de la partie de corps de tige (6;6A;6B) ;
(b) une étape de formation d'une partie poreuse (7;7B) de formation de la partie poreuse (7;7B) ; et
(c) une étape de liaison de liaison de la partie de corps de tige (6;6A;6B) à la partie poreuse (7;7B),
(d) dans lequel dans l'étape de formation de la partie poreuse (7;7B), la partie poreuse (7;7B) est formée en formant un composant autonome tubulaire en empilant des couches d'une poudre métallique biocompatible, et
(e) dans l'étape de liaison, la partie poreuse (7;7B) est liée à la partie de corps de tige (6;6A;6B) dans un état dans lequel la partie poreuse (7;7B) est appariée avec une partie proximale (11a) de la partie d'insertion (11;11B) ; **caractérisé en ce que**
(f) l'épaisseur (Ta) d'une partie proximale (21a) du corps poreux (21) est établie pour être plus grande que l'épaisseur (Tb) d'une partie distale (21b) du corps poreux (21) ;
(g1) dans lequel une multitude d'ouvertures internes (18a) sont formées dans la surface périphérique interne (7c) de la partie poreuse (7;7B) et une multitude d'ouvertures externes (18b) sont formées dans la surface périphérique externe (7d) de la partie poreuse (7;7A) ; et
(g2) dans lequel un rapport de surface totale (Pa) des ouvertures internes (18a) dans la surface périphérique interne (7c) est inférieur au rapport de surface totale (Pb) des ouvertures externes (18b) dans la surface périphérique externe (7d), dans lequel le rapport de surface totale (Pa) des ouvertures internes (18a) se rapporte au rapport de la surface totale de toutes les ouvertures internes (18a) à la surface de la surface périphérique interne (7c) de la partie poreuse (7B) et dans lequel le rapport de surface totale (Pb) des ouvertures externes (18b) se rapporte à la surface totale de toutes les ouvertures externes (18b) à la surface de la surface périphérique externe (7d) de la partie poreuse (7;7B).

13. Procédé de fabrication d'une tige de joint artificiel (4;4B) selon la revendication 12, dans lequel dans l'étape de liaison, la partie poreuse (7;7B) est liée à la partie d'insertion (11;11B) de manière à serrer la partie proximale (11a) de la partie d'insertion (11;11B).

14. Procédé de fabrication d'une tige de joint artificiel (4;4B) selon la revendication 12 ou 13,
dans lequel dans l'étape de liaison, la partie poreuse (7;7B) et la partie d'insertion (11;11B) sont liées par interposition d'une suspension épaisse entre la partie poreuse (7;7B) et la partie d'insertion (11;11B) et le chauffage de la suspension épaisse, ou en réalisant une jonction de diffusion sur la partie poreuse (7;7B) et la partie d'insertion (11;11B).

15. Procédé selon la revendication 12, dans lequel au moins certaines des ouvertures internes (18a) sont formées pour être en communication avec une ou plusieurs des ouvertures externes (18b).
